# EUROPEAN PATENT SPECIFICATION

(11) **EP 2 625 276 B1**
(45) Date of publication and mention of the grant of the patent: **28.03.2018**
(21) Application number: 11773939.1
(22) Date of filing: 07.10.2011
(51) Int. Cl.: C12N 15/82

(54) **CLOSTEROVIRUS-BASED NUCLEIC ACID MOLECULES AND USES THEREOF**
AUF CLOSTEROVIRUS BASIERENDE NUKLEINSÄUREMOLEKÜLE UND IHRE VERWENDUNG
MOLÉCULES D'ACIDE NUCLÉIQUE DÉRIVÉES D'UN CLOSTÉROVIRUS ET LEURS UTILISATIONS

(30) Priority: 08.10.2010 US 391333 P
(43) Date of publication of application: 14.08.2013
(73) Proprietor: Fraunhofer USA Inc., Newark, DE 19711-5449 (US)
(72) Inventor: PROKHNEVSKY, Alexei, Wilmington, DE 19808 (US); YUSIBOV, Vidadi, Havertown, PA 19083 (US); METT, Vadim, Elma, NY 14059-9450 (US)
(74) Representative: RatnerPrestia
(86) International application number: PCT/US2011/055365
(87) International publication number: WO 2012/048221

(56) References cited:
- EP-A1- 1 686 176
- V. V. PEREMYSLOV: "HSP70 homolog functions in cell-to-cell movement of a plant virus", PROCEEDINGS OF THE NATIONAL ACADEMY OF SCIENCES, vol. 96, no. 26, 21 December 1999 (1999-12-21), pages 14771-14776, XP55015320, ISSN: 0027-8424, DOI: 10.1073/pnas.96.26.14771
- GLEBA ET AL: "Viral vectors for the expression of proteins in plants", CURRENT OPINION IN BIOTECHNOLOGY, LONDON, GB, vol. 18, no. 2, 17 April 2007 (2007-04-17) , pages 134-141, XP022032092, ISSN: 0958-1669, DOI: 10.1016/J.COPBIO.2007.03.002 cited in the application

## Description

### FIELD OF THE INVENTION

The present invention relates generally to novel nucleic acid molecules for producing target polypeptides in plant cells. More specifically, the nucleic acid molecules comprise a minireplicon derived from a *Closteroviridae* virus and polynucleotides encoding the target polypeptides.

### BACKGROUND OF THE INVENTION

Production of recombinant proteins, including monoclonal antibodies (mAbs), vaccine antigens, enzymes, dual vaccines, fusion molecules and virus-like particles (VLPs), using plant viral vectors is an attractive alternative to traditional mammalian cell-based expression systems. Due to the high speed of virus replication, plant viral vectors have the potential to rapidly produce large quantities of foreign proteins.

A tobacco mosaic virus (TMV)-based vector is the most widely used vector for transient expression of plant-produced subunit vaccines and therapeutic proteins (Streatfield; Yusibov at al.; Gleba at al.; Rybicki). V.V Peremyslov: "HSP70 homolog functions in cell-to-cell movement of a plant virus", Proceedings of the National Academy of Sciences, vol 96, no. 26, 21. December 1999 (199-12-21), pages 14771-14776 discloses a beet yellows closterovirus (BYV) based vector expressing GFP protein linked to silencing suppressor gene HCPro from tobacco etch potyvirus (TEV) under the promotor of the capsid protein BYV. However, expression of high molecular weight proteins or co-expression of multiple polypeptide chains or proteins using TMV vectors is challenging. There remains a need for improved plant viral vectors for producing single large target proteins as well as simultaneously producing multiple target proteins in plants.

### SUMMARY OF THE INVENTION

The above problem was solved as recited in claims 1 and 8. Further embodiments of the invention are subject of the dependent sub-claims.

The disclosed subject matter of the present invention relates to novel nucleic acid molecules for producing target polypeptides in plant cells.

According to one aspect of the present invention, an isolated nucleic acid molecule for producing one or more target polypeptides in a plant cell is provided. The nucleic acid comprises a minireplicon derived from a *Closteroviridae* virus and one or more heterologous polynucleotides. The nucleic acid molecule is capable of replicating in the plant cell. The one or more heterologous polynucleotides encode the one or more target polypeptides. The nucleic acid molecule may further comprise a polynucleotide encoding one or more movement proteins derived from the *Closteroviridae* virus. The *Closteroviridae* virus may be *Beet yellows virus.*

The plant cell may be in a plant, a plant part, or a cell culture medium. The plant may be a whole growing plant. The plant part may be selected from the group consisting of leaves, stems, roots, floral tissues, seeds and petioles.

In one embodiment, the one or more target polypeptides comprise one or more subunits of a protein, and are capable of forming the protein in the plant cell. The protein may be an enzyme.

In another embodiment, the one or more target polypeptides comprise a first polypeptide and a second polypeptide, and the first polypeptide is capable of modifying the second polypeptide in the plant cell.

In yet another embodiment, the one or more target polypeptides comprise a first polypeptide and a second polypeptide, and the first polypeptide is capable of affecting expression of the second polypeptide in the plant cell. The first polypeptide may be a silencing suppressor.

In a further embodiment, the one or more target polypeptides comprise a first polypeptide and a second polypeptide, and the first polypeptide is capable of increasing production of the second polypeptide in the plant cell.

In yet a further embodiment, the one or more target polypeptides comprise a heavy chain and a light chain of an antibody, and are capable of forming the antibody in the plant cell.

The one or more target polypeptides may comprise an immunogenic polypeptide. The one or more target polypeptides comprise a polypeptide of at least 100 kD.

For each nucleic acid molecule of the present invention, a vector comprising the nucleic acid molecule is provided.

According to another aspect of the present disclosure, a method for producing one or more target polypeptides in a plant cell is provided. The method comprises (a) introducing a nucleic acid molecule into the plant cell; and (b) maintaining the plant cell under conditions permitting production of the one or more target polypeptides in the plant cell. The nucleic acid molecule comprises a minireplicon derived from a *Closteroviridae* virus and one or more heterologous polynucleotides. The nucleic acid molecule is capable of replicating in the plant cell. The one or more heterologous polynucleotides encode the one or more target polypeptides. The one or more target polypeptides comprise a polypeptide of at least 100 kD. The method may further comprise purifying at least one of the one or more target polypeptides from the plant cell. The nucleic acid molecule may further comprise a polynucleotide encoding one or more movement proteins derived from the *Closteroviridae* virus. The *Closteroviridae* virus may be *Beet yellows virus.*

In the production method of the present disclosure, the plant cell may be in a plant, a plant part, or a cell culture medium. The plant may be a whole growing plant. The plant part may be selected from the group consisting of leaves, stems, roots, floral tissues, seeds and petioles.

A composition comprising at least one of the one or more target polypeptides produced by the method of the present invention is provided. A method of treating a subject in need of at least one of the one or more target polypeptides produced thereby is also provided. The treatment method comprises administering to the subject an effective amount of a pharmaceutical composition comprising the at least one of the one or more target polypeptides.

In one embodiment, the one or more target polypeptides comprise one or more subunits of a protein. In the production method, the maintaining conditions further permit production of the protein in the plant cell. A composition comprising the protein produced thereby is provided. A method of treating a subject in need of the protein is also provided. The treatment method comprises administering to the subject an effective amount of a pharmaceutical composition comprising the protein. The protein may be an enzyme.

In another embodiment, the one or more target polypeptides comprise a first polypeptide and a second polypeptide. In the production method, the maintaining conditions further permit modifying, affecting expression, and/or increasing production of the second polypeptide by the first polypeptide in the plant cell. A composition comprising the second polypeptide is provided. A method of treating a subject in need of the second polypeptide is also provided. The treatment method comprises administering to the subject an effective amount of a pharmaceutical composition comprising the second polypeptide. The first polypeptide may be a silencing suppressor.

In yet another embodiment, the one or more target polypeptides comprise a heavy chain and a light chain of an antibody. In the production method, the maintaining conditions further permit production of the antibody in the plant cell. A composition comprising the antibody is provided. A method of treating a subject in need of the antibody is also provided. The treatment method comprises administering to the subject an effective amount of a pharmaceutical composition comprising the antibody.

In a further embodiment, the one or more target polypeptides comprise an immunogenic polypeptide. A composition comprising the immunogenic polypeptide produced thereby is provided. A method for inducing an immune response in a subject is also provided. The induction method comprises administering to the subject an effective amount of a pharmaceutical composition comprising the immunogenic polypeptide. A method for inducing a protective immune response against a pathogen in a subject is further provided. The protective induction method comprises administering to the subject an effective amount of a pharmaceutical composition comprising the immunogenic polypeptide. The immunogenic polypeptide is derived from the pathogen.

### BRIEF DESCRIPTION OF THE DRAWINGS

Figure 1 is a diagram illustrating the organization of the BYV genome. L-Pro - papain-like leader proteinase; Met, Hel, and Pol - methyltransferase, RNA helicase, and RNA-dependent RNA polymerase domains of the replicase, respectively; p6 - 6 kD protein; Hsp70h - a Hsp70 homolog; p64 - 64 kD protein; CPm and CP - minor and major capsid proteins, respectively; p20 and p21 - 20 and 21 kD proteins, respectively.
Figure 2 is a diagram illustrating a T-DNA region of a BYV-based launch vector encoding Hc and Lc of an anti-PA antibody. 35S - *Cauliflower mosaic virus* 35S promoter; NOS - *Nopaline synthase* terminator; LB and RB - left and right borders of T-DNA, respectively.
Figure 3A shows *N. benthamiana* leaves systematically infected with the BYV-based launch vector encoding Hc and Lc of an anti-PA antibody. Figure 3B shows Western blot analysis of Hc, Lc and total anti-PA IgG expression in the systematically infected *N. benthamiana* leaves at 30, 32, 34, 36 and 39 days post infiltration (dpi). The maximum expression of total anti-PA IgG was observed on day 34, with 53 mg/kg of fresh leaf weight. *Standards, ng of total human IgG.
Figure 4 is a diagram illustrating a T-DNA region of a T-DNA-based BYV minireplicon vector for expression of Hc and Lc of an anti-PA antibody. The Hc and Lc genes are under the control of the BYV CP promoter and the GLRaV2 CP promoter, respectively.
Figure 5 is a diagram illustrating a T-DNA region of a T-DNA-based BYV minireplicon vector for expression of Hc and Lc of the anti-PA antibody. The Hc and Lc genes are under the control of the BYV CP promoter and BYSV CP promoter, respectively.
Figure 6A-B shows Western blot analysis of Hc and Lc expression in *N*. *benthamiana* leaves systematically infected by a modified miniBYV vector at 5 and 7 dpi, respectively. Figure 6C shows calculated amounts of Lc and Hc expression in the systematically infected *N. benthamiana* leaves at 5, 7 and 9 dpi. Lanes: 100, 50 and 25 (ng) of the human mAb standard. Q3, #1, Q3, #2, Q3, #3, Q4, #1, Q4, #2, Q4, #3 - different clones of the miniBYV replicon carrying Hc and Lc of the anti-PA mAb.
Figure 7 is a diagram illustrating a T-DNA region of a miniBYV replicon vector for expression of the anthrax Protective Antigen 83 (PA83) under the control of the BYV promoter.
Figure 8A shows Western blot analysis of PA83 expression in systematically infected *N. benthamiana* leaves at 5, 7 and 9 dpi. Figure 8B shows the amounts of total protein (TP) and total soluble protein (TSP) expression in the systematically infected *N*. *benthamiana* leaves at 5, 7 and 9 dpi. Lanes 1-9: 1, PA standard, 50 ng; 2, PA standard, 25 ng; 3, PA standard, 10 ng; 4, pCB-miniBYV-PA83, TP; 5, pCB-miniBYV-PA83, TSP; 6, pGR-D4-PA83, TP*; 7, pGR-D4-PA83, TSP*; 8, pClean 238-PA83, TP; 9, pClean238-PA83, TSP. * Without a silencing suppressor P1HcPro.
Figure 9 is a diagram illustrating a T-DNA region of a miniBYV replicon vector for co-expression of target protein Pfs48 and an enzyme capable of modifying the target protein.
Figure 10 shows co-expression of target protein Pfs48 and an enzyme PNGaseF capable of deglycosylating target protein Pfs48. Detection was made using anti-His antibody (A) and anti-FLAG antibody (B), respectively.
Figure 11 is a diagram illustrating a T-DNA region of a miniBYV replicon vector for expression of three open reading frames (ORFs) for three different targets using a combination of strong and weak closteroviral promoters (i.e., BYV CP promoter, GLRaV2 CP promoter, and BYSV CP promoter).
Figure 12A-H shows the nucleic acid sequence of a T-DNA region of a BYV launch vector (SEQ ID NO: 1) according to some embodiments of the disclosed subject matter. A BYV sequence (upper case) with multiple cloning sites (bold) along with the 35S promoter (lower case) and the NOS terminator (lower case italic) are introduced between the left border (LB) and right border (RB) of the T-DNA sequence (underline). The multiple cloning sites (bold) include PacI (14,254-14,261 bp), AscI (14,262-14,269), BsrGI (14,270-14,275), NheI (14,276-14,281) and FseI (14,285-14,292).

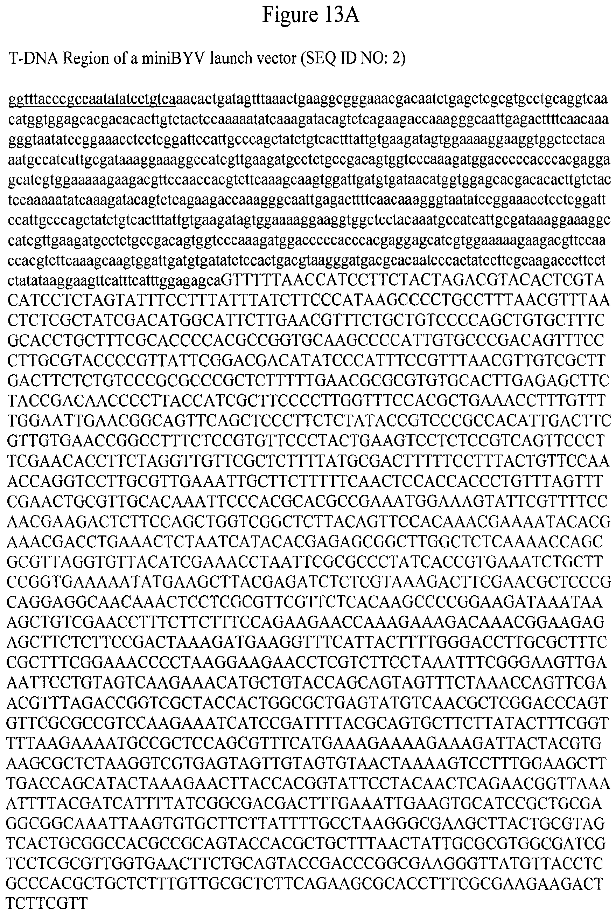

Figure 13A-E shows the nucleic acid sequence of a T-DNA region of a miniBYV launch vector (SEQ ID NO: 2) according to some embodiments of the disclosed subject matter. A miniBYV sequence (upper case) with multiple cloning sites (bold) along with the 35S promoter with a dual enhancer (lower case) and the NOS terminator (lower case italic) are introduced between the left border (LB) and right border (RB) of the T-DNA sequence (underline). The multiple cloning sites (bold) include BamHI (10,278-10,285), PacI (10,286-10,293), AscI (10,294-10,301), BsrGI (10,302-10,307), NheI (10,308-10,313) and FseI (10,317-10,324).
Figure 14 shows the nucleic acid sequences of (A) BYV CP promoter (SEQ ID NO: 3), (B) BYSV CP promoter (SEQ ID NO: 4), and (C) GLRaV2 CP promoter (SEQ ID NO: 5).
Figure 15 shows (A) an amino acid sequence of Lc of an anti-PA antibody with the PR1a signal peptide from *Nicotiana tavacum* on the N-terminus (underline) and (SEQ ID NO: 6), and (B) a corresponding nucleic acid sequence with a TGA stop codon (underline) (SEQ ID NO: 7).
Figure 16 shows (A) an amino acid sequence of Hc of an anti-PA antibody with the PR1a signal peptide from *Nicotiana tavacum* on the N-terminus (underline) (SEQ ID NO: 8) and (B) a corresponding nucleic acid sequence with a TGA stop codon (underline) (SEQ ID NO: 9).
Figure 17 shows (A) an amino acid sequence of PA83 with the PR1a signal peptide from *Nicotiana tavacum* on the N-terminus (underline) and 6His (bold) for purification and KDEL (italic) as an ER retention signal on the C-terminus (SEQ ID NO: 10), and (B) a corresponding nucleic acid sequence with a TGA stop codon (underline) (SEQ ID NO: 11).
Figure 18 shows (A) an amino acid sequence of Pfs48 with the PR1a signal peptide from *Nicotiana tavacum* on the N-terminus (underline) and 6His (bold) for purification and KDEL (italic) as an ER retention signal on the C-terminus (SEQ ID NO: 12) and (B) a corresponding nucleic acid sequence with a TGA stop codon (underline) (SEQ ID NO: 13).
Figure 19 shows (A) an amino acid sequence of PNGaseF with the PR1a signal peptide from *Nicotiana tavacum* on the N-terminus (underline) and a FLAG tag (bold) for detection and KDEL (italic) as an ER retention signal on the C-terminus (SEQ ID NO: 14) and (B) a corresponding nucleic acid sequence with a TGA stop codon (underline) (SEQ ID NO: 15).

### DETAILED DESCRIPTION OF THE INVENTION

The present invention is based on the discovery that novel nucleic acid molecules comprising a *Beet yellows virus* (BYV) minireplicon can be used to produce a single or multiple heterologous target polypeptides in a plant cell. The BYV minireplicon may be used for production of therapeutically active proteins, subunit vaccines, protein adjuvants, enzymes, monoclonal antibodies (mAbs) and virus-like particles (VLP).

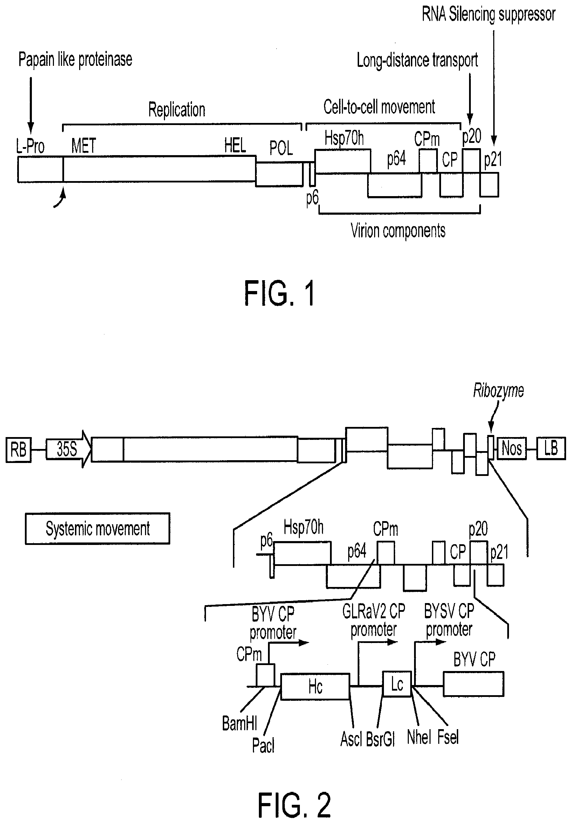

BYV is a member of the alphavirus supergroup of positive-strand RNA viruses belonging to the genus *Closterovirus,* family *Closteroviridae.* The 15.5 kb monopartite genome of BYV encodes 8 open reading frames (ORFs) (Fig. 1). Three groups of proteins are recognized in the BYV genome. The first group of proteins is responsible for virus replication, and includes methyltransferase (Met), helicase (Hel), and RNA polymerase (Pol) (ORF 1A and 1B). The second group of proteins is responsible for the virus cell-to-cell movement (ORFs 2-6), and includes P6, HSP70h, CP, CPm and p64. The knockout of any one of these proteins results in an arrest of the virus cell-to-cell movement. The third group of proteins includes viral structural components such as Hsp70H, CP, CPm, p64 and p20 (ORFs 3-7). p20 also known as the viral long distance transport factor. p21- the BYV silencing suppressor (ORF 8).

BYV contains a replication gene block which covers more than 50% of the BYV genome and includes genes necessary for BYV replication. The BYV replication gene block is formed by the domain of papain-like leader proteinase (L-Pro), methyltransferase (Met), helicase-like domain region of viral replicase (Hel), and RNA-depended RNA polymerase (Pol). RNA-depended RNA polymerase is expressed from +1 frameshift. A larger replication protein which contains methyltransferase, helicase, and polymerase is produced in smaller quantities compared to the methyltransferase-helicase polyprotein due to the low frequency of frameshifting (Fig. 1). Flexious BYV virions are ∼1300 nm in length and ∼12 nm in diameter, and contain five structural proteins. The major capsid protein (CP) encapsidates ∼95% of the virion body. A short virion tail which is necessary for the BYV cell-to-cell and systemic movement contains minor CP (CPm); Hsp70h, a homolog of cellular heat shock proteins; p64, a 64 kD protein with unknown functions; and p20, a long distance transport factor. Other proteins of BYV are p6, a small transmembrane protein required for BYV cell-to-cell movement and localized in the endoplasmic reticulum of host cell; and p21, a BYV silencing suppressor involved in binding of short interfering RNA.

The term "protein" used herein refers to a biological molecule comprising amino acid residues. A protein may comprise one or more polypeptides. Each polypeptide may be a subunit of protein. For example, the protein may be an antibody consisting of two Hc and two Lc. The protein may be in a native or modified form, and may exhibit a biological function when its polypeptide or polypeptides are properly folded or assembled.

The term "polypeptide" used herein refers to a polymer of amino acid residues with no limitation with respect to the minimum length of the polymer. Preferably, the polypeptide has at least 20 amino acids. A polypeptide may be a full-length protein, or a fragment or variant thereof.

The term "fragment" of a protein as used herein refers to a polypeptide having an amino acid sequence that is the same as a part, but not all, of the amino acid sequence of the protein. Preferably, a fragment of a protein retains the same function as the protein.

The term "variant" of a protein as used herein refers to a polypeptide having an amino acid that is the same as the amino acid sequence of the protein except having at least one modification, for example, glycosylation, phosphorylation, a deletion, an addition or a substitution. The variant may have an amino acid at least 80%, 90%, 95%, or 99%, preferably at least 90%, more preferably at least 95%, identical to the amino acid sequence of the protein. Preferably, a variant of a protein retains the same function as the protein.

The term "derived from" used herein refers to the origin or source, and may include naturally occurring, recombinant, unpurified or purified molecules.

According to one aspect of the present disclosure, a nucleic acid molecule for producing one or more target polypeptides in a plant cell is provided. The nucleic acid molecule comprises a minireplicon derived from a *Closteroviridae* virus and one or more heterologous polynucleotides, and is capable of replicating in the plant cell. The one or more heterologous polynucleotides encode the one or more target polypeptides.

A *Closteroviridae* virus may be any virus in the family of *Closteroviridae.* For example, the *Closteroviridae* virus may be *Beet Yellows virus, Grapevine leafroll-associated virus* 2 (GLRaV2), *Beet yellows stunt virus* (BYSV), *Citrus tristeza virus* (CTV), *Carrot yellow leaf virus* (CYLV), or *Lettuce infectious yellows* virus *(LIYV).* Preferably, the *Closteroviridae* virus is *Beet yellows virus.*

A plant cell may be a cell in any plants, plant parts (e.g., leaves, stems, roots, floral tissues, seeds and petioles) or cell culture media. The plant may be a whole growing plant. The cell culture media may be any media suitable for growing plant cells, preferably in suspension. The plant cell is preferably susceptible to infection by a *Closteroviridae* virus. More preferably, the plant cell is susceptible to BYV infection. The plant cell is preferably suitable for expression of a target polypeptide. For example, the plant cell may be cells in *N. benthamiana* leaves. Other suitable plants include *Nicotiana clevelandii, Beta vulgaris, Spinacia oleracea, Brassica* spp, *Lactuca sativa, Pisum sativum, Nicotiana tabacum, Plantago lanceolata, Tetragonia tetragonioides, Montia perfoliata, Beta vulgari, Spinacia oleracea, Stellaria media, Brassica* spp., *Lactuca sativa, Pisum sativum, Nicotiana tabacum, Plantago lanceolata, Montia perfoliata, Tetragonia tetragonioides, Chenopodium foliosum,* and *Nicotiana benthamiana.*

A minireplicon derived from a *Closteroviridae* virus is a polynucleotide, comprising a nucleic acid sequence encoding only proteins, each of which corresponds to a natural viral replication protein of the *Closteroviridae* virus required for replication of the virus. Each encoded protein exhibits the same function as its corresponding natural viral replication protein, and may have an amino acid sequence at least 80%, 85%, 90%, 95%, or 99%, preferably at least 95%, more preferably at least 99%, most preferably 100%, identical to that of its corresponding natural viral replication protein. The minireplicon may be generated from the genome of the *Closteroviridae* virus by deleting nucleic acid sequences, including genes, not required for the replication of the virus. For example, a BYV minireplicon may be a replication gene block formed by the L-Pro domain, methyltransferase (Met), helicase (Hel) and RNA-dependent RNA polymerase (Pol) as shown in Fig. 1.

For each nucleic acid molecule of the present invention, a vector comprising the nucleic acid is provided. The vector may include border sequences of a bacterial transfer DNA at either end, and be situated in a bacterial transfer DNA, to allow for delivery of the nucleic acid of the present invention into a plant cell. Specifically, the vector may comprise one or more nucleic acid sequences derived from a Ti plasmid of a binary vector (e.g., right border (RB) and left border (LB) in Fig. 2). Such a vector, including elements of a Ti plasmid and a viral vector, is also called a launch vector. This vector may also be used for co-expression of a target polynucleotide of interest with a protein, such as a silencing suppressor or a modifying enzyme such as PNGaseF, to modify, affect expression and/or increase production the target polypeptide. The protein may facilitate maturation or accumulation of the target polypeptide.

A heterologous polynucleotide is a polynucleotide that is foreign, not native, to the *Closteroviridae* virus and the target cell. It may comprise a nucleic acid sequence encoding a target polypeptide, which may be expressed in a plant cell.

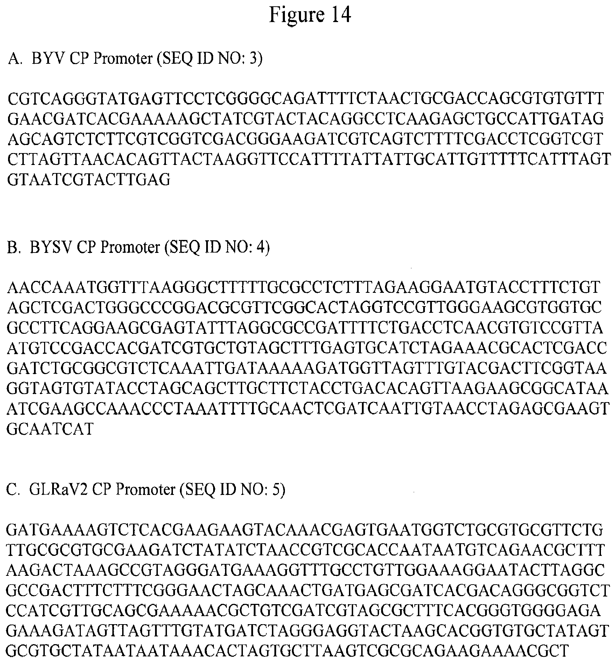

In the nucleic acid molecule of the present invention, each heterologous polynucleotide encodes a target polypeptide, and may be operatively linked to a viral promoter derived from any virus in the family *Closteroviridae.* Examples of suitable viral promoters include BYV promoters, *Grapevine leafroll-associated* virus 2 (GLRaV2) promoters, and/or *Beet Yellows stunt* virus (BYSV) promoters. Preferably, polynucleotides in the same nucleic acid molecule are operably linked to different viral promoters. Exemplary viral promoters include BYV CP promoter (SEQ ID NO: 3; Fig. 14), BYSV CP promoter (SEQ ID NO: 4; Fig. 14), and GLRaV2 CP promoter (SEQ ID NO: 5; Fig. 14).

The nucleic acid molecule of the present disclosure may further comprise a polynucleotide encoding one or more movement proteins derived from the *Closteroviridae* virus. Examples of the movement proteins include p6, Hsp70h, p64, CPm, CP and p20 of BYV. The movement proteins may enhance the movement of the nucleic acid molecule from one plant cell to another and cause systemic spread of the nucleic acid molecule, thereby increasing plant production level of the target polypeptide encoded by the heterologous polynucleotide by, for example, at least 1%, 5%, 10%, 15%, 20%, 30%, 40%, 50%, 60%, 70%, 80%, 90%, 95%, 100%, 200%, 500% or 1000%.

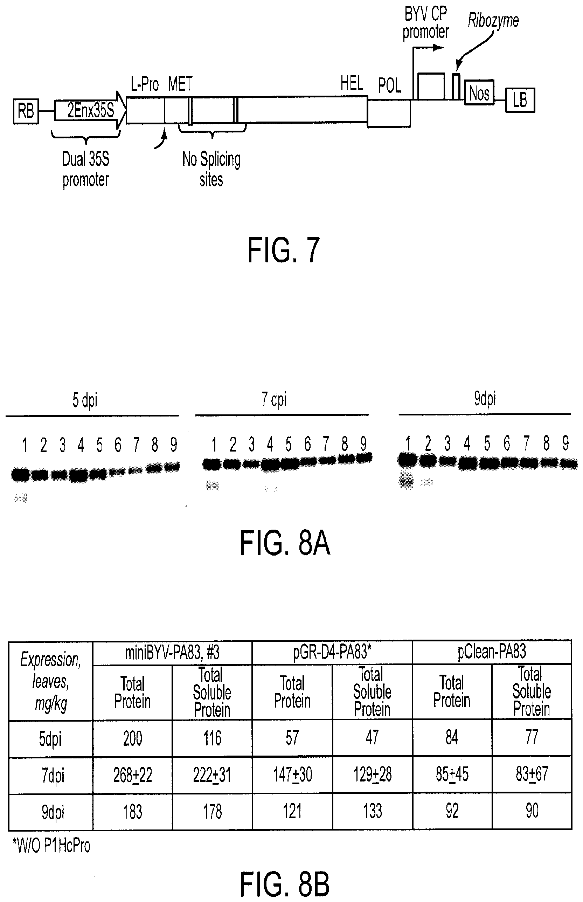

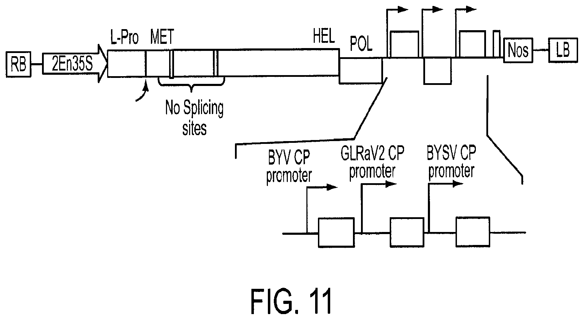

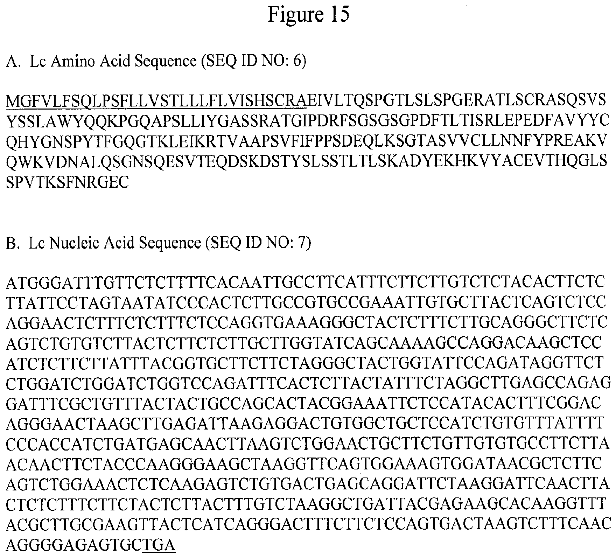

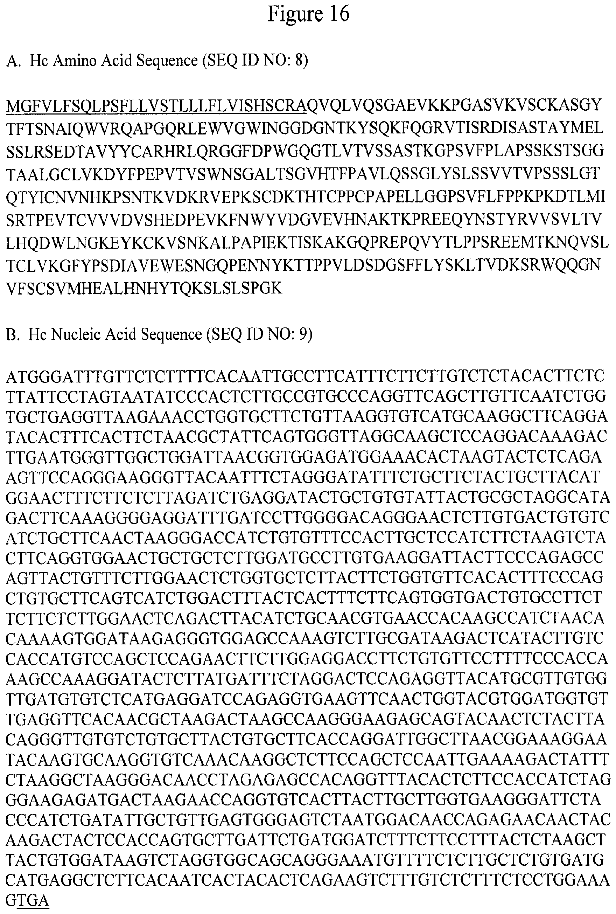

The nucleic acid molecule of the present disclosure may comprise one (e.g., Fig. 7), two (e.g., Figs. 2, 4, 5, and 9), three (e.g., Fig. 11) or more heterologous polynucleotides, each of which encodes a target polypeptide. Preferably, the nucleic acid molecule comprises two or more heterologous polynucleotides encoding two or more target polypeptides, and the target polypeptides are expressed from the same minireplicon of the nucleic acid molecule within a plant cell. The target polypeptide may constitute a subunit of a protein. The target polypeptides may be capable of forming a protein such as an enzyme or antibody. For example, the nucleic acid molecule may comprise two heterologous polynucleotides encoding Hc (Fig. 16) and Lc (Fig. 15) of an antibody. In some embodiments, the nucleic acid may encode two target polypeptides, in which a first target polypeptide is capable of modifying, affecting expression and/or increasing production of a second target polypeptide in a plant cell. The first target polypeptide may facilitate maturation of the second polypeptide, which becomes biologically active. The first target polypeptide may also facilitate accumulation of the second polypeptide.

A target polypeptide may be any polypeptide capable of forming or becoming a functional protein (e.g., an enzyme or antibody) or a vaccine candidate. A target polypeptide may be of any size. It may have at least 6, 10, 50, 100, 200, 300, 400, 500, 750, or 1000 amino acids, preferably at least 100 amino acids, more preferably at least 500 amino acids, most preferably at least 750 amino acids. It may also be at least 10, 20, 50, 75, 100, 125, 150, or 200 kD, preferably at least about 100 kD, more preferably at least 125 kD, most preferably at least 150 kD.

A target polypeptide may be immunogenic. It may comprise one or more epitopes (linear and/or conformational) that are capable of stimulating the immune system of a subject to make a humoral and/or cellular antigen-specific immune response. A humoral immune response refers to an immune response mediated by antibodies produced by B lymphocytes, or B cells, while a cellular immune response refers to an immune response mediated by T lymphocytes, or T cells, and/or other white blood cells. In general, a B-cell epitope contains at least about 5 amino acids but can be 3-4 amino acids, while a T-cell epitope includes at least about 7-9 amino acids and a helper T-cell epitope includes at least 12-20 amino acids. A target polypeptide may be derived from a protein (e.g., a surface protein or toxin subunit) of a pathogenic organism or pathogen.

A "subject" may be an animal. For example, the animal may be an agricultural animal (e.g., horse, cow and chicken) or a pet (e.g., dog and cat). Preferably, the subject is a mammal. Most preferably, the subject is a human. The subject may be a male or female. The subject may also be a newborn, child or adult. The subject may have suffered a disease or medical condition.

For each of the nucleic acid molecules of the present disclosure, a method for producing one, two, three or more target polypeptides in a plant cell is provided. The method comprises (a) introducing the nucleic acid molecule into a plant cell; and (b) maintaining the plant cell under conditions permitting production of the target polypeptide(s) in the plant cell. The nucleic acid molecule comprises a minireplicon derived from a *Closteroviridae* virus, and is capable of replicating in the plant cell. The nucleic acid molecule further comprises one, two, three or more heterologous polynucleotides, each of which encodes a target polypeptide. The plant cell may be a cell in a plant, a plant part (e.g., leaf, stem, root, floral tissue, seed or petiole) or a cell culture medium. The plant may be a whole growing plant. Preferably, the plant cell is in a plant leaf.

The nucleic acid molecule of the present invention may be introduced into a plant cell using techniques known in the art. For example, the nucleic acid molecule may be delivered into the plant cell via infiltration, bombardment, or manual inoculation. The nucleic acid molecule could be used as a part of an inducible system activated by, for example, chemical, light or heat shock. Preferably, the nucleic acid molecule is introduced into a plant cell via infiltration.

For production of a target polypeptide by a plant or plant cells infected by a vector of the present invention, the infected plant or plant cells are maintained under conditions permitting for the production. Such conditions include suitable temperature, humidity, pressure, timing, and illumination. As described below in Examples 2 and 4-6, nucleic acid molecules of the present invention have been introduced into plant cells, which were maintained under conditions permitting production of one or two polypeptides, and such production was observed. The production method may further comprise purifying at least one of the target polypeptide(s) from the plant. The target polypeptide may be purified from the plant using techniques known in the art. For example, the target polypeptide may be purified from the plant using an antibody or a receptor capable of binding the target polypeptide. The purification process may comprise extraction of the target from *N. benthamiana* using extraction buffer. After low speed centrifugation, supernatant may be clarified by filtration and used for chromatography. The purified product may be at least 10%, 20%, 30%, 40%, 50%, 60%, 70%, 80%, 90%, 95% or 99%, preferably at least 50%, more preferably at least 75%, most preferably at least about 95%, pure. The target polypeptide may be used in a crude plant extract. For example, an industrial enzyme expressed in plant tissues according to the present invention, and a crude plant extract containing the enzyme may be used in an industrial process.

In the production method according to the present disclosure, the *Closteroviridae* virus may be any virus in the family of *Closteroviridae.* Examples of the *Closteroviridae* virus include BYV, *Grapevine leafroll-associated* virus 2 (GLRaV2), and *Beet Yellows stunt* virus (BYSV). Preferably, the *Closteroviridae* virus is BYV. The vector may further comprise a polynucleotide encoding one or more movement proteins derived from the *Closteroviridae* virus. The movement proteins may enhance movement of the heterologous polynucleotide from one plant cell to another plant cell, and thereby increase plant production level of the target polypeptide encoded by the heterologous polynucleotide by, for example, at least 1%, 5%, 10%, 15%, 20%, 30%, 40%, 50%, 60%, 70%, 80%, 90%, 95%, 100%, 200%, 500% or 1000%.

For each production method of the present disclosure, a composition comprising the one, two, three or more target polypeptides produced thereby is provided. Also provided is a method of treating a subject in need of the one, two, three or more target polypeptides. The treatment method comprises administering to the subject an effective amount of a pharmaceutical composition comprising the target polypeptide(s). The pharmaceutical composition may further comprise a pharmaceutically acceptable carrier or diluents. Suitable carriers or diluents are known in the art and include, but are not limited to, saline, buffered saline, mannitol, L-histidine, polysorbate 80, dextrose, water, glycerol, ethanol, and combinations thereof. The pharmaceutical composition may optionally contain an adjuvant. The pharmaceutical composition may have a pH of 4.0-10.0, preferably 5.6-7.0.

The term "an effective amount" refers to an amount of a pharmaceutical composition comprising the target polypeptide(s) required to achieve a stated goal (e.g., treating a subject in need of the target polypeptide(s), or inducing an immune response in a subject). The effective amount of the pharmaceutical composition comprising the target polypeptide(s) may vary depending upon the stated goal, the physical characteristics of the subject, the nature and severity of the need of the target polypeptide(s), the existence of related or unrelated medical conditions, the nature of the target polypeptide(s), the composition comprising the target polypeptide(s), the means of administering the composition to the subject, and the administration route. A specific dose for a given subject may generally be set by the judgment of a physician. The pharmaceutical composition may be administered to the subject in one or multiple doses.

The target polypeptide(s) may be formulated in a pharmaceutical composition of the present disclosure. The pharmaceutical composition may be formulated for administration to a subject via various routes, for example, oral, sublingual, intranasal, intraocular, rectal, transdermal, mucosal, topical or parenteral administration.

In a production method according to the present disclosure, the one, two, three or more target polypeptides may be one or more subunits of a protein, and the maintaining conditions may further permit production of the protein in the plant cell. A composition comprising the protein produced thereby is provided. A method of treating a subject in need of the protein is also provided. The treatment method comprises administering to the subject an effective amount of a pharmaceutical composition comprising the protein. The protein may be an enzyme.

In a production method according to the present disclosure, a first target polypeptide may be capable of modifying, affecting expression, and/or increasing production of a second target polypeptide in the plant cell, and the maintaining conditions may further permit modifying, affecting expression, and/or increasing production of the second target polypeptide by the first target polypeptide in the plant cell. A composition comprising the second target polypeptide produced thereby is provided. A method of treating a subject in need of the modified target polypeptide is also provided. The treatment method comprises administering to the subject an effective amount of a pharmaceutical composition comprising the polypeptide.

In a production method according to the present invention, the target polypeptides may be Hc and Lc of an antibody, and the maintaining conditions may further permit production of the antibody in the plant cell. A composition comprising the antibody produced thereby is provided. A method of treating a subject in need of the antibody is also provided. The treatment method comprises administering to the subject an effective amount of a pharmaceutical composition comprising the antibody.

In a production method according to the present invention, the target polypeptide may be immunogenic. A composition comprising the immunogenic target polypeptide is provided. A method for inducing an immune response in a subject is also provided. The immunogenic method comprises administering to the subject an effective amount of a pharmaceutical composition comprising the immunogenic target polypeptide. Where the immunogenic target polypeptide is derived from a pathogen, the immunogenic method may be used for inducing a protective immune response against the pathogen in a subject by administering to the subject an effective amount of a pharmaceutical composition comprising the immunogenic target polypeptide. The pathogen may be an intracellular or extracellular pathogen.

### Example 1. Construction of BYV-based launch vector for monoclonal antibody expression

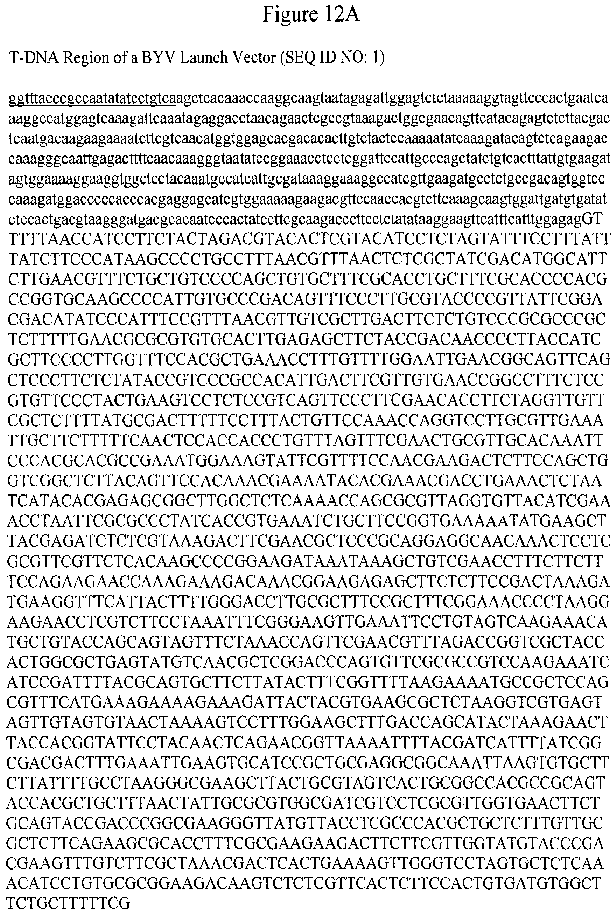

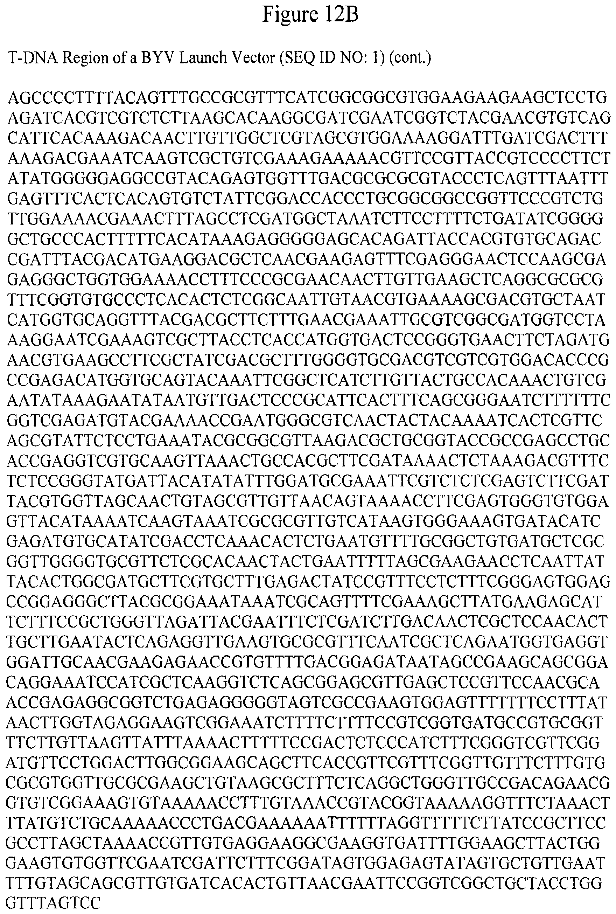

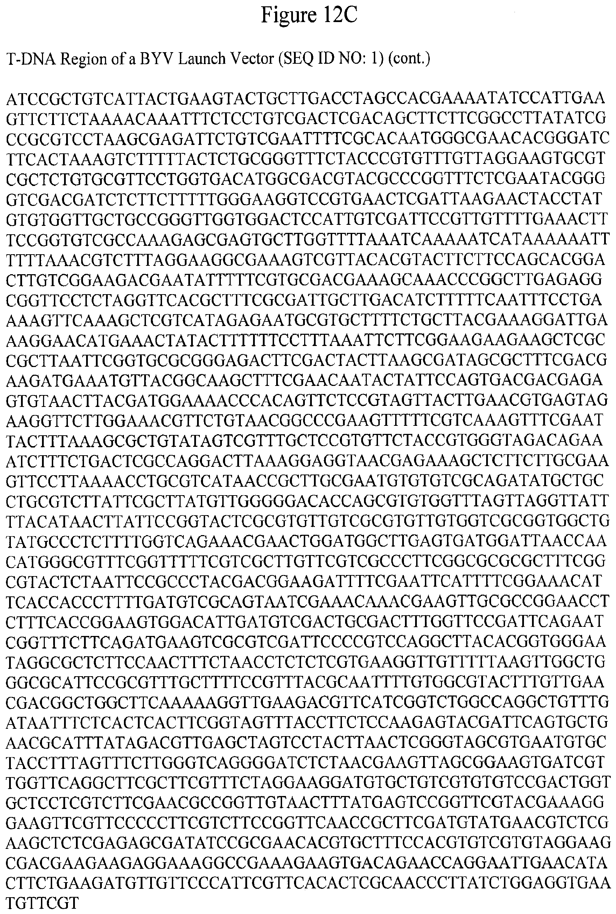

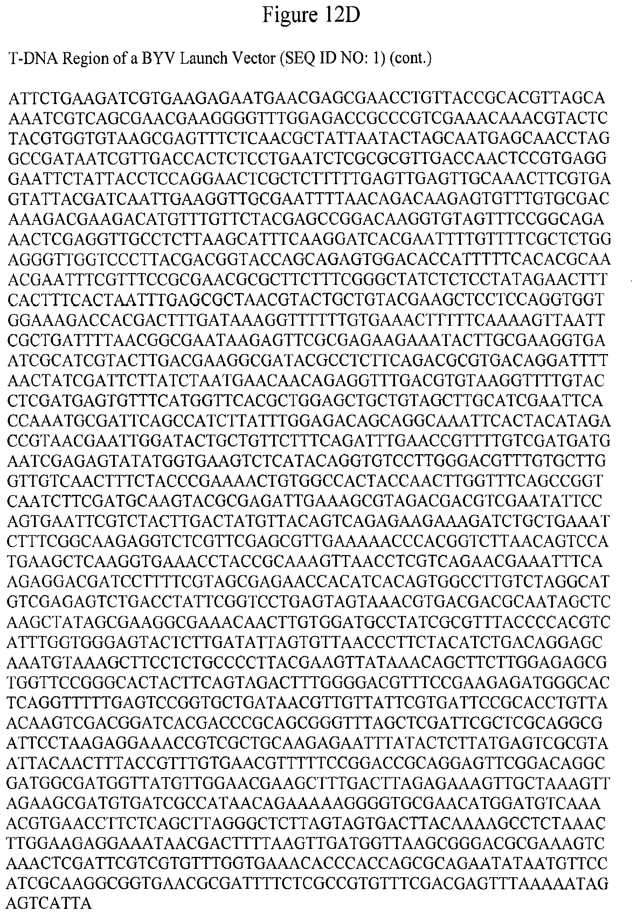

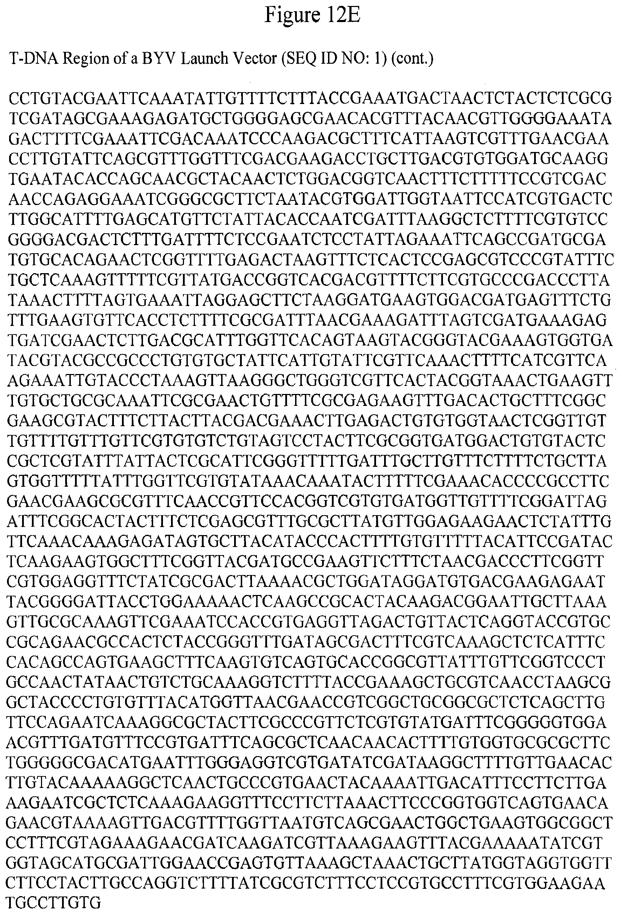

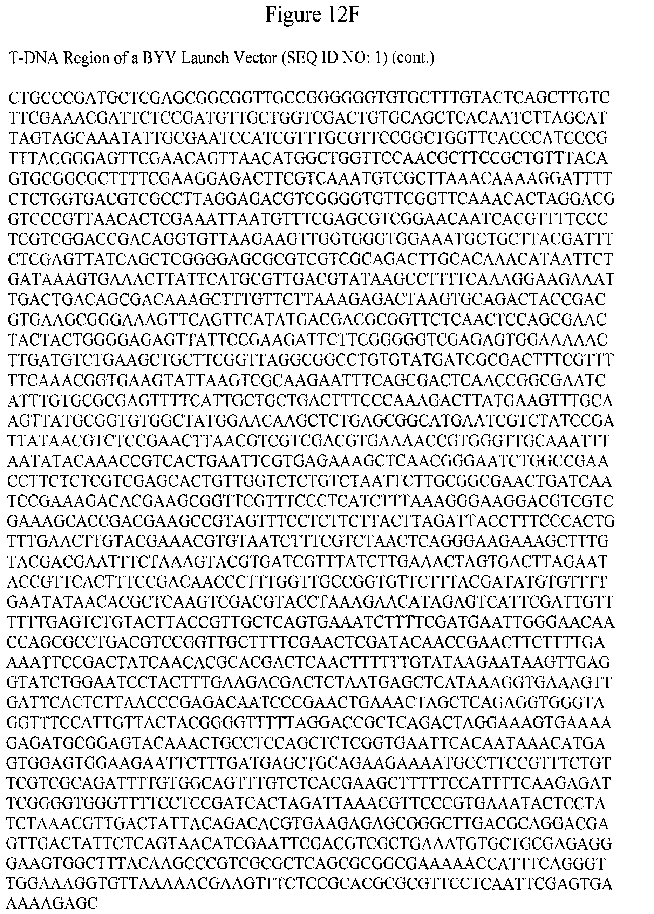

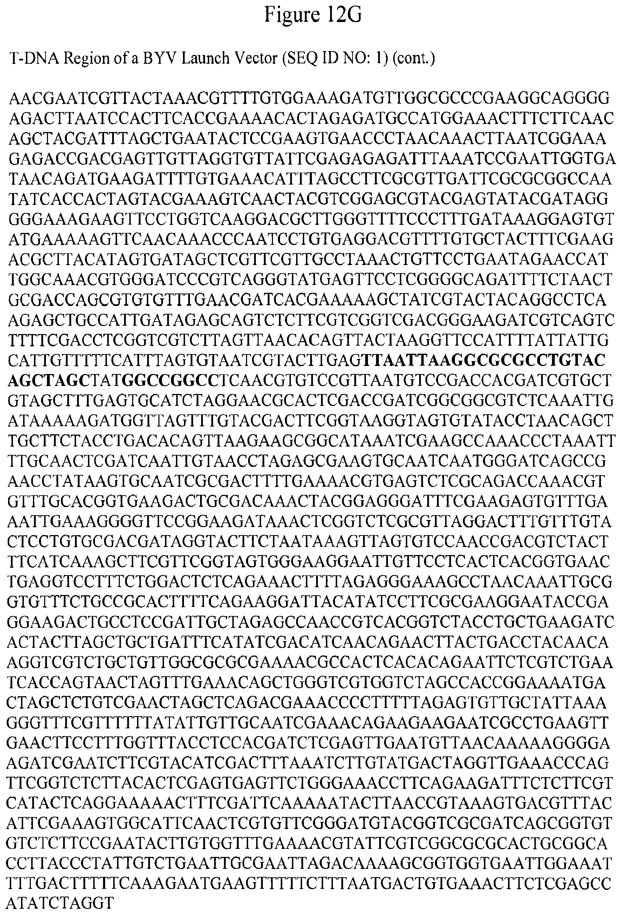

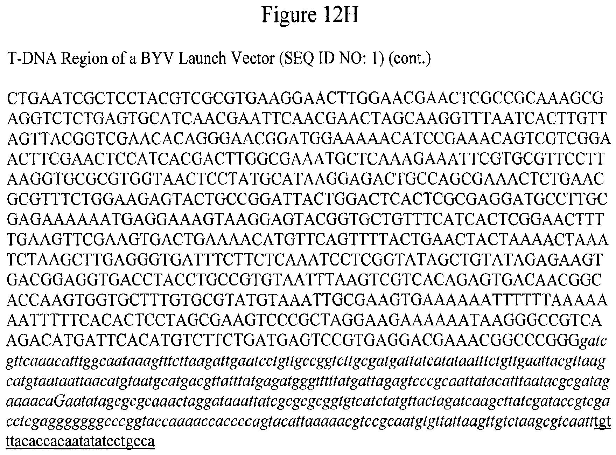

A BYV-based launch vector for simultaneous expression of two target polypeptides within the same host cell was constructed (Fig. 2). The BYV launch vector was used as a carrier for expression of two ORFs, Hc and Lc of a mAb against PA of anthrax. For cloning of two foreign genes (Lc and Hc of the anti-PA mAb), a multiple cloning site (MCS) was introduced into the BYV genome between the CPm and CP coding sequences (SEQ ID NO: 1, Fig. 12). The MCS contains 5 restriction sites, PacI/AscI/BsrGI/NheI/FseI, in addition to the native BamHI restriction site. After inserting the MCS, two heterologous closteroviral CP promoters were introduced into the BYV genome: the GLRaV2 CP promoter and the BYSV CP promoter (Fig. 2). As a result, the sequences of Hc (SEQ ID NO: 9; Fig. 16) and Lc (SEQ ID NO: 7; Fig. 15) of the anti-PA mAb were cloned under the control of the BYV CP and the GLRaV2 CP promoters, respectively. Meanwhile, the BYSV CP promoter drives the BYV CP (Fig. 2). The resulting construct pCB-BYV-PA-HcLc was transformed into *Agrobacterium tumefaciens* strain GV3101.

### Example 2. Expression of anti-PA mAb in systemically BYV-infected leaves

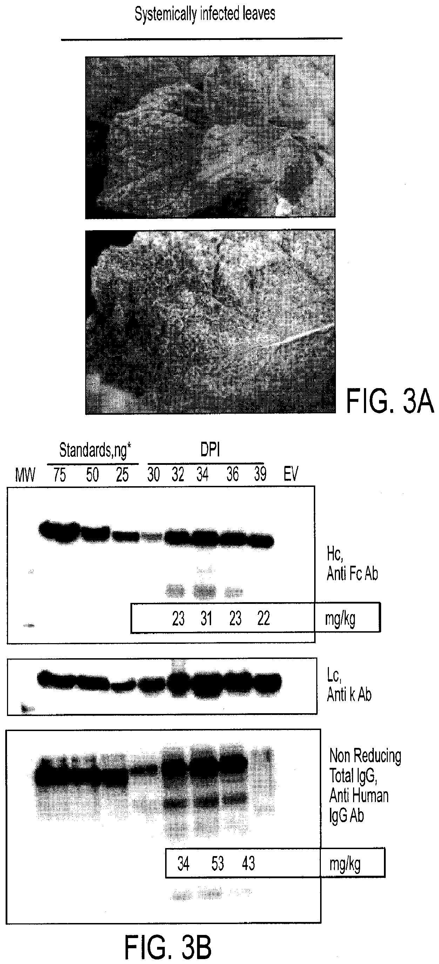

To confirm the stability of the virus and the expression and assembly of the anti-PA mAb, 5-week-old *N. benthamiana* leaves were manually co-infiltrated by overnight-grown (at 28 °C) cultures of agrobacteria carrying a BYV vector encoding Hc and Lc of the anti-PA mAb and agrobacteria carrying a binary vector encoding a silencing suppressor P1HcPro from *Turnip mosaic virus* (Kasschau et al, 2003), at a ratio of 1.0:0.2 OD₆₀₀. After 30 dpi, systemic symptoms of the BYV infection were observed (Fig. 3A). In particular, the infected leaves showed clearing veins as the systemic symptoms. Samples were collected from systemically infected leaves at 30, 32, 34, 36 and 39 dpi.

To demonstrate the expression of Lc and Hc of the anti-PA mAb, Western blot analysis was employed (Fig. 3B). To assess the Hc and Lc expression levels, horseradish peroxidase (HRP)-conjugated goat anti-human Hc and Lc antibodies (Bethyl Laboratories Inc.) were used at dilution of 1:5000 and 1:2000, respectively. A purified anti-PA mAb was used to serve as a positive control. The expression levels were calculated using GeneGnome5 gel imaging and analysis systems from Synoptics Ltd. Using the same technique under non-reducing conditions, the expression level of the assembled anti-PA mAb was calculated. The maximum expression level was determined to be 53 mg/kg of fresh leaf weight at 34 dpi.

### Example 3. Construction of a T-DNA-based BYV minireplicon

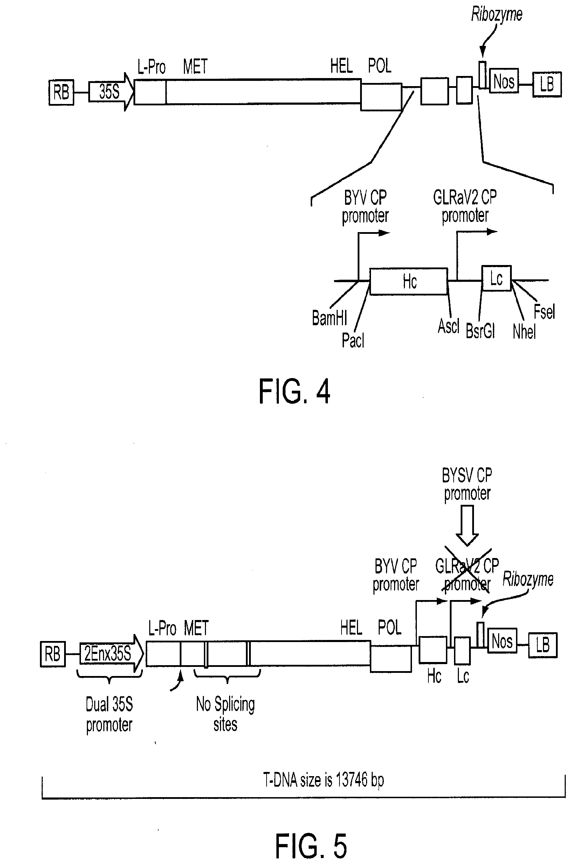

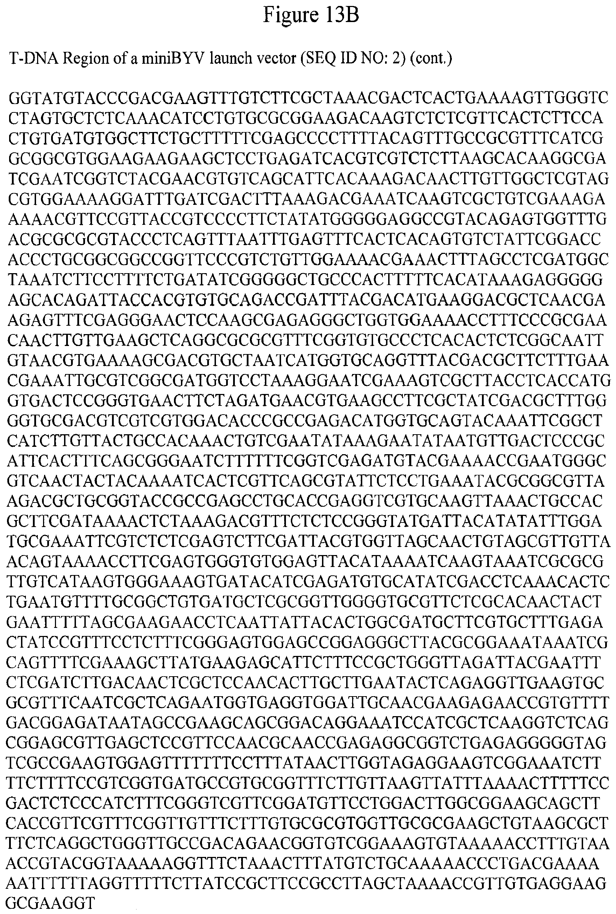

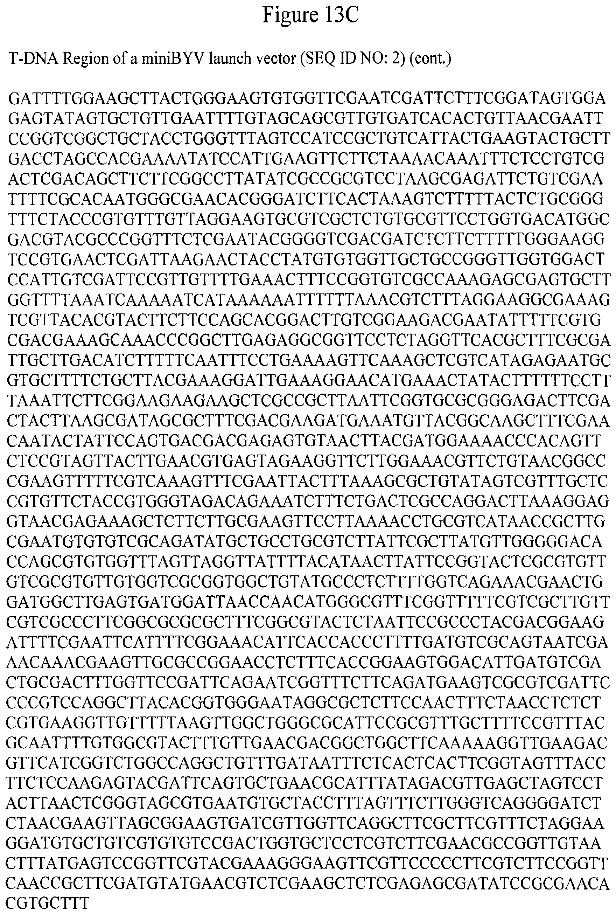

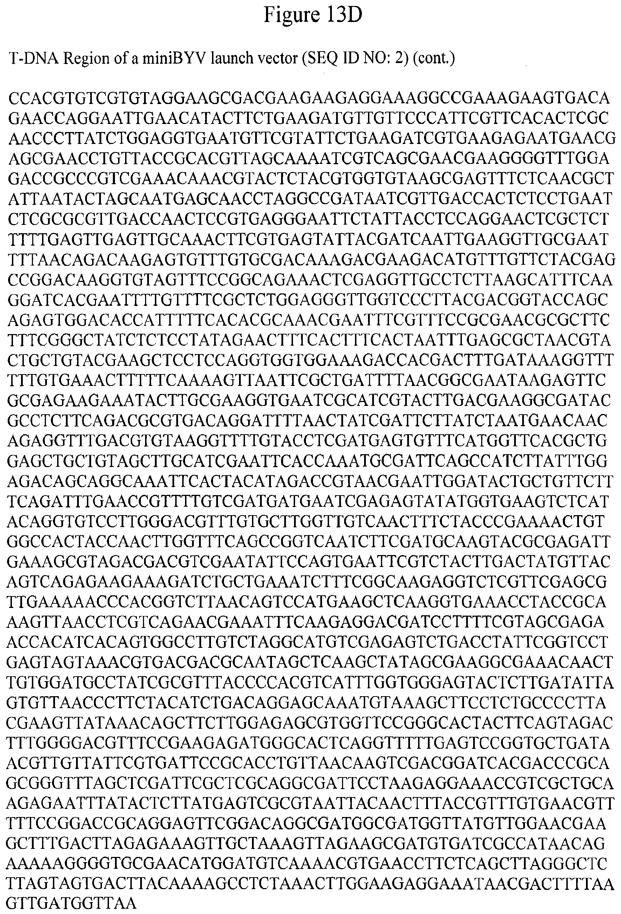

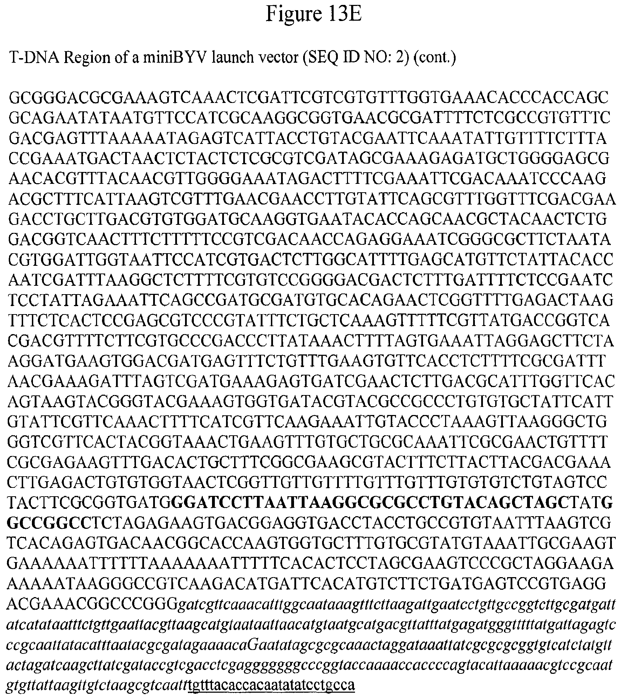

To decrease the production time and increase the antibody expression level, a T-DNA-based BYV minireplicon (miniBYV) was engineered by removing all genes which are not necessary for viral replication from the BYV-based launch vector as described in Example 1 (Figs. 1, 2 and 4). Using the native BamHI restriction site, the same MCS as the one inserted into the whole-length BYV-based vector in Example 1 was introduced into the miniBYV replicon (SEQ ID NO: 2; Fig. 13) (Figs. 2 and 4). This strategy allowed for using heterologous closteroviral subgenomic promoters to express two foreign genes from a single miniBYV replicon. In addition, two closteroviral promoters, the BYV CP promoter and GLRaV2 CP promoter, were introduced to drive Hc (SEQ ID NO: 9; Fig. 16) and Lc (SEQ ID NO: 7; Fig. 15) of the anti-PA mAb, respectively.

To prevent splicing and increase the efficiency of viral invasiveness, the canonical splicing sites were removed from the viral replicase sequence. To increase the transcription level of miniBYV RNA, *Cauliflower mosaic virus* 35S promoter with a dual enhancer was inserted upstream of the 5' end of the miniBYV sequence (Fig. 4).

To increase the amount of the synthesized initial transcript, the 35S promoter with dual enhancers was introduced upstream of the miniBYV sequence to generate a modified miniBYV vector (Fig. 5). The weak GLRaV2 CP promoter was replaced by the strong BYSV CP promoter. The BYV replicase was analyzed for the presence of the canonical splicing sites using the SplicePredictor software from the Center for Bioinformatics and Biological Statistics, Iowa State University. *Arabidopsis* was used as a splicing site model. To avoid potential splicing, the high-scoring canonical acceptor splicing site within the BYV replicase was mutated by substituting the nucleotide 2219 (GenBank Accession No. AF 190581) from A to C, which was confirmed by sequencing. This also allowed for knocking out the donor site in the position 3606. The final size of T-DNA insert carrying miniBYV with Hc and Lc of the mAb was 13,746 bp (Fig. 5). Example 4. Expression of anti-PA mAb in leaves systemically infected with the modified miniBYV vector (pCB-BYV-Hc-Lc)

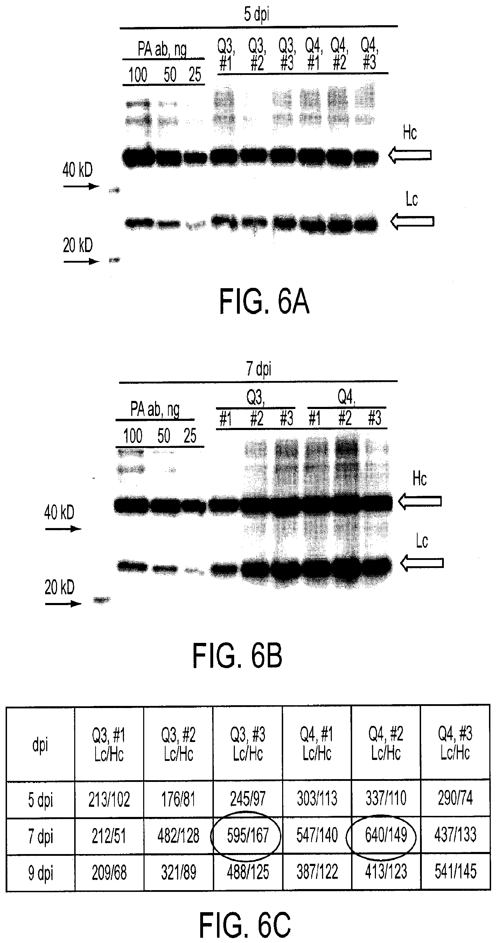

To examine the expression level of Hc and Lc of the mAb from the modified miniBYV vector, 5-week-old *N. benthamiana* plants were infiltrated as described in Example 2 using two clones (Q3 and Q4, confirmed by sequencing) and three agro colonies were collected for each clone. To confirm that both Hc and Lc were expressed, the leaf disks were taken at 5, 7 and 9 dpi, analyzed by Western blotting and calculated as described above. The results demonstrate a three-fold increase in the expression level of Hc and Lc of the anti-PA mAb using the modified miniBYV launch vector carrying two strong promoters (Fig. 6) and suggest that the miniBYV vector can be used for antibody production.

### Example 5. Expression of a large protein (PA83) using the miniBYV vector

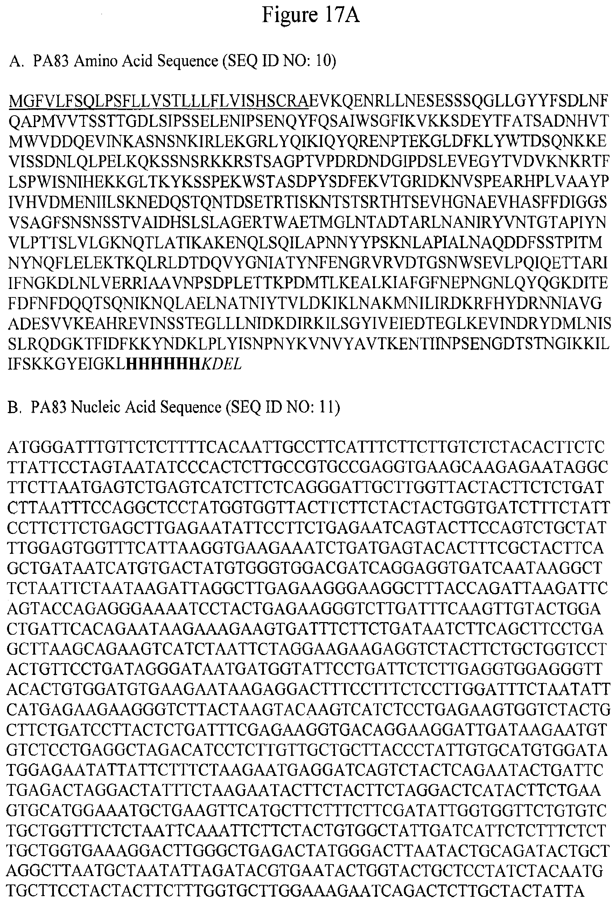

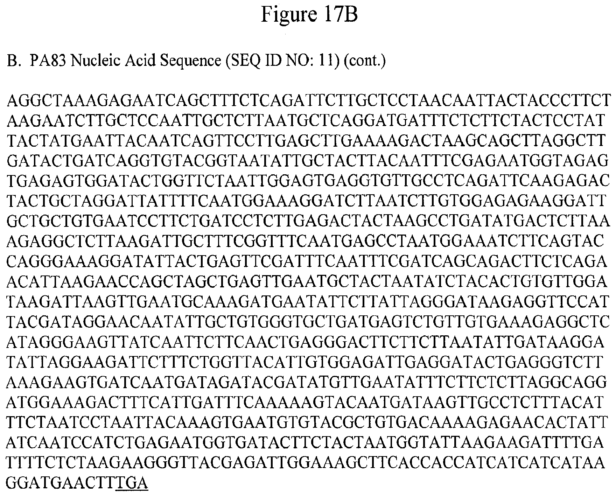

To investigate a possibility of using the miniBYV vector for expressing large proteins, anthrax Protective Antigen 83 (PA83, GenBank accession no. M22589) with a molecular weight of 83 kD from *Bacillus anthracis* was used. The sequence of PA83 with added PR-1a signal peptide from *Nicotiana tabacum,* 6xHistidine affinity purification tag and an endoplasmic reticulum (ER) retention signal (KDEL) (SEQ ID NO: 11; Fig. 17) was plant optimized by GENEART Inc. (Germany) and cloned into the miniBYV vector using PacI/NheI restriction sites (Fig. 7). The final construct was confirmed by sequencing. A binary vector carrying the miniBYV-PA83 vector was transformed into the GV3101 strain of agrobacteria. The expression level of PA83 from the miniBYV was compared to other vectors such as TMV-based launch vector pGR-D4-PA83 and a regular binary vector pClean283-PA83 carrying a dual 35S promoter with a TEV leader. Five-month-old *N. benthamiana* leaves were manually infiltrated as described above.

The total protein (TP) and total soluble protein (TSP) expression levels at 5, 7 and 9 dpi were analyzed (Fig. 8A). The infiltration and analysis were repeated three times for 7 dpi to confirm the calculated numbers. As shown in Fig. 8B, the highest expression level was observed for PA83 using the miniBYV vector at 7 dpi (268 ± 22 mg/kg) with 83% solubility. The expression level was at least two times higher for the miniBYV replicon compared to the regular binary vector (pClean) and more than 60% higher compared to the TMV-based vector (D4) (Fig. 8).

Using an immobilized metal ion adsorption chromatography (IMAC) column, the PA83 protein expressed from the miniBYV replicon was purified. The purification process consisted of an extraction of the target from *N. benthamiana* leaf tissue with extraction buffer (50 mM Na-Phosphate pH 8.0, 500 mM NaCI, 20 mM Imidazole and 1 mM diethylcarbamic acid [DIECA]) at the 3:1 v/w ratio. After clarification, PA83 was captured using Ni-IMAC by employing the Chelating Sepharose Big Beads resin. The target was eluted with buffer contacting 300 mM Imidazole (obtained by mixing 60% of Buffer B [50 mM Na-Phosphate pH 7.5, 500 mM NaCl, 500 mM Imidazol] with 40% of Buffer A [50 mM Na-Phosphate pH 7.5, 500 mM NaCl]). Western blot analysis showed that final recovery of PA83 expressed from the miniBYV vector after IMAC purification was about 72%.

### Example 6. Co-expression of a target protein and an enzyme using the miniBYV vector

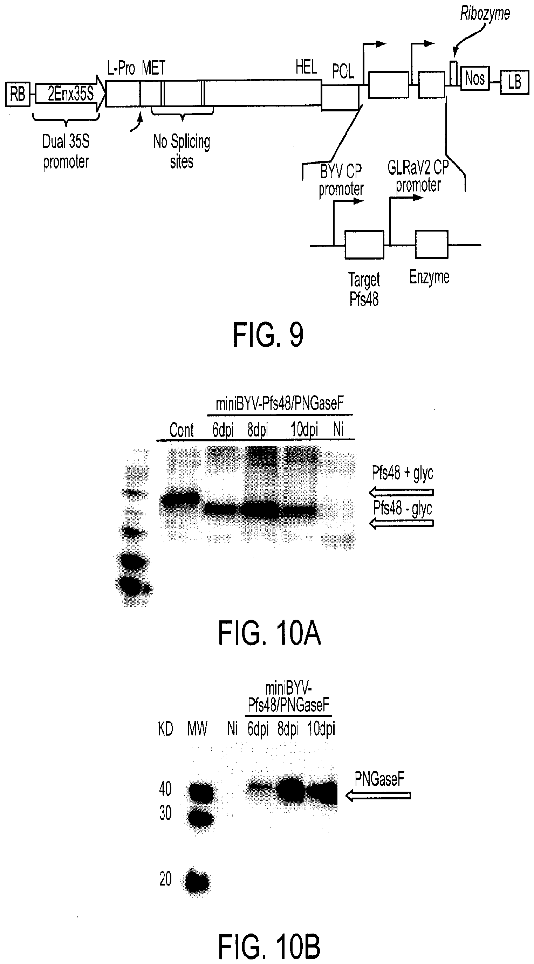

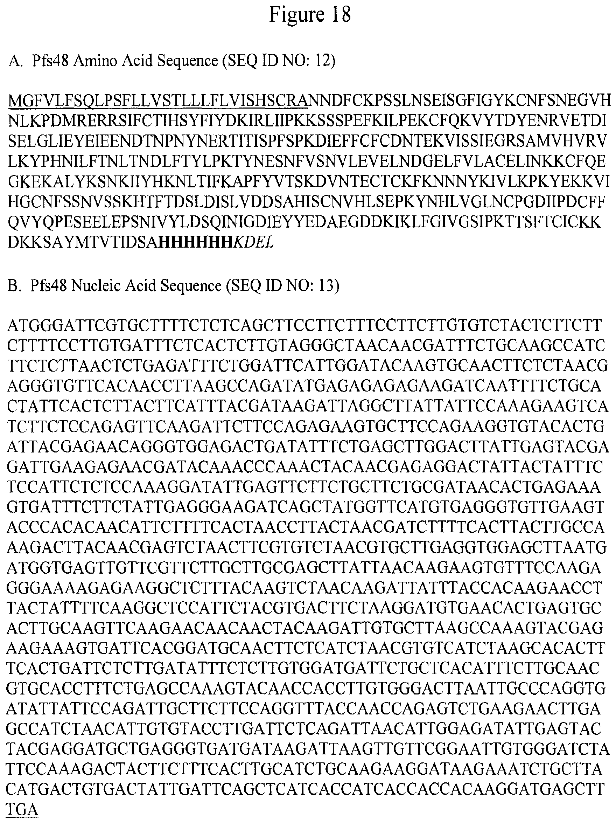

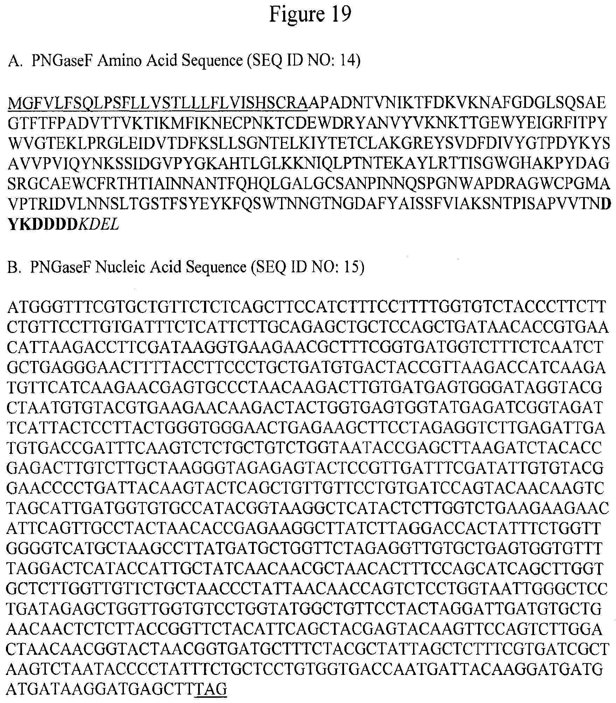

To explore a possibility of using the miniBYV vector for co-expression of a target protein and a modifying enzyme, malaria vaccine candidate protein Pfs48 (from protozoa parasite *Plasmodium falciparum,* accession # AAL74351) and endoglycosidase F from *Elizabethkingia meningoseptica* (PNGaseF, accession no AAA24932) were used. Again, a plant GENEART-optimized sequence of Pfs48 with the PR-1a peptide at the N-terminus and the 6xHis-tag and KDEL on the C-terminus of the protein (SEQ ID NO: 13; Fig. 18) was used. For PNGaseF, the PR-1a peptide on N-terminus of the protein and FLAG-tag and KDEL ER retention signal on the C-terminus (SEQ ID NO: 15; Fig. 19) were used. Both sequences were cloned into the miniBYV vector as shown in Fig. 9. A weak GLRaV2 CP promoter was used to control PNGaseF because the enzyme toxic to the plant tissue when expressed at a higher level.

The expression level of Pfs48 was assessed using a mouse anti-histidine mAb at a 1:2000 dilution (anti-4xHis, Qiagen Inc.). Fig. 10A shows that the electrophoretic mobility of the glycosylated Pfs48 (control, which was expressed w/o PNGaseF) was different (less mobility) compared to the non-glycosylated form of Pfs48 that was co-expressed with PNGaseF. The expression of PNGaseF was confirmed by Western blotting using a rabbit anti-FLAG primary mAb (Sigma) and a goat anti-rabbit secondary Ab (Biorad) (Fig. 10B). These results verify identical compartmentalization of both the target (Pfs48) and the enzyme (PNGaseF), which is probably inside of the virus replication complex. The results also verify that the PNGaseF acted on the Pfs48.

### References

Dolja VV. Beet yellows virus: the importance of being different. Mol Plant Pathol. 2003 Mar 1; 4(2):91-8.
Giritch A, Marillonnet S, Engler C, van Eldik G, Botterman J, Klimyuk V, Gleba Y. Rapid high-yield expression of full-size IgG antibodies in plants coinfected with noncompeting viral vectors. Proc Natl Acad Sci U S A. 2006, 103(40): 14701-6.
Gleba Y, Klimyuk V, Marillonnet S. Viral vectors for the expression of proteins in plants. Curr Opin Biotechnol. 2007, 18(2):134-41.
Hull AK, Criscuolo CJ, Mett V, Groen H, Steeman W, Westra H, Chapman G, Legutki B, Baillie L, Yusibov V. Human-derived, plant-produced monoclonal antibody for the treatment of anthrax. Vaccine. 2005, 18; 23(17-18):2082-6
Kasschau KD, Carrington JC. Long-distance movement and replication maintenance functions correlate with silencing suppression activity of potyviral HC-Pro. Virology. 2001, 285(1):71-81.
Mett V, Chichester JA, Stewart ML, Musiychuk K, Bi H, Reifsnyder CJ, Hull AK, Albrecht MT, Goldman S, Baillie LW, Yusibov V. A non-glycosylated, plant-produced human monoclonal antibody against anthrax protective antigen protects mice and non-human primates from B. anthracis spore challenge. Hum Vaccin. 2011, 7 Suppl:183-90
Musiychuk K, Stephenson N, Bi H, Farrance CE, Orozovic G, Brodelius M, Brodelius P, Horsey A, Ugulava N, Shamloul AM, Mett V, Rabindran S, Streatfield SJ, Yusibov V. A launch vector for the production of vaccine antigens in plants. Influenza Other Respi Viruses. 2007, 1(1):19-25
Peng C., Peremyslov VV, Mushegian AR, Dawson WO, Dolja VV. Functional specialization and evolution of leader proteinases in the family of Closteroviridae. J. Virol. 2001, 75(24): 12153-60.
Prokhnevsky AI, Peremyslov VV, Napuli AJ, Dolja VV. Interaction between long-distance transport factor and Hsp70-related movement protein of Beet yellows virus. J Virol. 2002, 76(21):11003-11.
Roy G, Weisburg S, Foy K, Rabindran S, Mett V, Yusibov V. Co-expression of multiple target proteins in plants from a tobacco mosaic virus vector using a combination of homologous and heterologous subgenomic promoters. Arch Virol. 2011, [Epub ahead of print]
Roy G, Weisburg S, Rabindran S, Yusibov V. A novel two-component Tobacco mosaic virus-based vector system for high-level expression of multiple therapeutic proteins including a human monoclonal antibody in plants. Virology. 2010, 405(1):93-9.
Rybicki EP. Plant-made vaccines for humans and animals. Plant Biotechnol J. 2010, 8(5):620-37
Streatfield SJ. Approaches to achieve high-level heterologous protein production in plants.Plant Biotechnol J. 2007, 5(1):2-15.
Yusibov V, Streatfield SJ, Kushnir N. Clinical development of plant-produced recombinant pharmaceuticals: vaccines, antibodies and beyond. Hum Vaccin. 2011, 7(3):313-21.

## Claims

1. A nucleic acid molecule for producing two or more target polypeptides in a plant cell, comprising a nucleic acid sequence derived from a *Beet Yellows* virus (BYV) and two or more heterologous target polynucleotides, wherein the nucleic acid molecule replicates in the plant cell, wherein each of the two or more heterologous target polynucleotides is under the control of a separate promoter and encodes one of the two or more target polypeptides, and wherein the nucleic acid sequence derived from the BYV encodes proteins consisting of the domain of BYV papain-like leader proteinase (L-Pro), BYV methyltransferase, helicase-like domain region of BYV replicase, and BYV RNA-dependent RNA polymerase.

2. The nucleic acid molecule of claim 1, wherein the plant cell is in a plant, a plant part, or a cell culture medium.

3. The nucleic acid molecule of any of claims 1 to 2, wherein the two or more target polypeptides comprise two or more subunits of a protein, and wherein the two or more target polypeptides are capable of forming the protein in the plant cell.

4. The nucleic acid molecule of claim 3, wherein the protein is an enzyme.

5. The nucleic acid molecule of any of claims 1 to 2, wherein the two or more target polypeptides comprise a heavy chain and a light chain of an antibody, and wherein the two or more target polypeptides are capable of forming the antibody in the plant cell.

6. The nucleic acid molecule of any of claims 1 to 5, wherein the two or more target polypeptides comprise one or more immunogenic polypeptides.

7. The nucleic acid molecule of any of claims 1 to 5, wherein two or more of the target polypeptides comprise a polypeptide of at least 100 kD.

8. A method for producing two or more target polypeptides in a plant cell, comprising
(a) introducing the nucleic acid molecule of claim 1 into the plant cell, and
(b) expressing the two or more target polypeptides in the plant cell.

9. The method of claim 8, further comprising purifying at least one of the two or more target polypeptides from the plant cell.

## Patentansprüche

1. Nucleinsäuremolekül zum Produzieren von zwei oder mehr Zielpolypeptiden in einer Pflanzenzelle, das eine von einem Rübenvergilbungsvirus (*Beet Yellows* Virus BYV) abgeleitete Nucleinsäuresequenz und zwei oder mehr heterologe Zielpolynucleotide beinhaltet, wobei sich das Nucleinsäuremolekül in der Pflanzenzelle repliziert, wobei jedes der zwei oder mehr heterologen Zielpolynucleotide unter der Kontrolle eines separaten Promotors steht und eines der zwei oder mehr Zielpolypeptide kodiert, und wobei die von dem BYV abgeleitete Nucleinsäuresequenz Proteine kodiert, die aus der Domäne von BYV-Papain-artiger Leitproteinase (L-Pro), BYV-Methyltransferase, einer Helikase-artigen Domänenregion von BYV-Replikase und BYV RNA-abhängiger RNA-Polymerase bestehen.

2. Nucleinsäuremolekül nach Anspruch 1, wobei die Pflanzenzelle in einer Pflanze, einem Pflanzenteil oder einem Zellkulturmedium ist.

3. Nucleinsäuremolekül nach einem der Ansprüche 1 bis 2, wobei die zwei oder mehr Zielpolypeptide zwei oder mehr Untereinheiten eines Proteins beinhalten und wobei die zwei oder mehr Zielpolypeptide das Protein in der Pflanzenzelle bilden können.

4. Nucleinsäuremolekül nach Anspruch 3, wobei das Protein ein Enzym ist.

5. Nucleinsäuremolekül nach einem der Ansprüche 1 bis 2, wobei die zwei oder mehr Zielpolypeptide eine schwere Kette und eine leichte Kette eines Antikörpers beinhalten und wobei die zwei oder mehr Zielpolypeptide den Antikörper in der Pflanzenzelle bilden können.

6. Nucleinsäuremolekül nach einem der Ansprüche 1 bis 5, wobei die zwei oder mehr Zielpolypeptide ein oder mehrere immunogene Polypeptide beinhalten.

7. Nucleinsäuremolekül nach einem der Ansprüche 1 bis 5, wobei zwei oder mehr der Zielpolypeptide ein Polypeptid von wenigstens 100 kD beinhalten.

8. Verfahren zum Produzieren von zwei oder mehr Zielpolypeptiden in einer Pflanzenzelle, das Folgendes beinhaltet:
(a) Einführen des Nucleinsäuremoleküls aus Anspruch 1 in die Pflanzenzelle und
(b) Exprimieren der zwei oder mehr Zielpolypeptide in der Pflanzenzelle.

9. Verfahren nach Anspruch 8, das ferner das Reinigen von wenigstens einem der zwei oder mehr Zielpolypeptide aus der Pflanzenzelle beinhaltet.

## Revendications

1. Molécule d'acide nucléique permettant de produire deux ou plusieurs polypeptides cibles dans une cellule végétale, qui comprend une séquence d'acide nucléique dérivée d'un virus de la jaunisse de la betterave (BYV) et deux ou plusieurs polynucléotides hétérologues cibles, où la molécule d'acide nucléique se reproduit dans la cellule végétale, où chacun des deux ou plusieurs polynucléotides hétérologues cibles est sous le contrôle d'un promoteur distinct et code pour l'un des deux ou plusieurs polypeptides cibles et où la séquence d'acide nucléique dérivée du BYV code pour des protéines consistant en le domaine L-Pro (pour Leader protéinase) de type papaïne du BYV, la méthyltransférase du BYV, une région du domaine de type hélicase de la réplicase du BYV et l'ARN-polymérase ARN-dépendante du BYV.

2. Molécule d'acide nucléique de la revendication 1, où la cellule végétale est dans une plante, un fragment d'une plante ou un milieu de culture cellulaire.

3. Molécule d'acide nucléique de l'une quelconque des revendications 1 ou 2, où les deux ou plusieurs polypeptides cibles comprennent deux ou plusieurs sous-unités d'une protéine et où les deux ou plusieurs polypeptides cibles sont capables de former la protéine dans la cellule végétale.

4. Molécule d'acide nucléique de la revendication 3, où la protéine est une enzyme.

5. Molécule d'acide nucléique de l'une quelconque des revendications 1 ou 2, où les deux ou plusieurs polypeptides cibles comprennent une chaîne lourde et une chaîne légère d'un anticorps et où les deux ou plusieurs polypeptides cibles sont capables de former l'anticorps dans la cellule végétale.

6. Molécule d'acide nucléique de l'une quelconque des revendications 1 à 5, où les deux ou plusieurs polypeptides cibles comprennent un ou plusieurs polypeptides immunogènes.

7. Molécule d'acide nucléique de l'une quelconque des revendications 1 à 5, où deux ou plusieurs des polypeptides cibles comprennent un polypeptide d'au moins 100 kD.

8. Procédé de production de deux ou plusieurs polypeptides cibles dans une cellule végétale comprenant
(a) l'introduction de la molécule d'acide nucléique de la revendication 1 dans la cellule végétale et
(b) l'expression des deux ou plusieurs polypeptides cibles dans la cellule végétale.

9. Procédé de la revendication 8, qui comprend en outre la purification d'au moins un des deux ou plusieurs polypeptides cibles produits dans la cellule végétale.


### SEQUENCE LISTING

<110> Fraunhofer USA Inc.
   Prokhnevsky, Alex
   Yusibov, Vidadi
   Mett, Vadim
<120> CLOSTEROVIRUS-BASED NUCLEIC ACID MOLECULES AND USES THEREOF
<130> FCMB-103WO
<150> 61/384,561
   <151> 2010-10-08
<160> 15
<170> PatentIn version 3.5
<210> 1
   <211> 16800
   <212> DNA
   <213> Artificial Sequence
<220>
   <223> Synthetic
<400> 1
<210> 2
   <211> 10933
   <212> DNA
   <213> Artificial Sequence
<220>
   <223> Synthetic
<400> 2
   ggtttacccg ccaatatatc ctgtcaaaca ctgatagttt aaactgaagg cgggaaacga      60
   caatctgagc tcgcgtgcct gcaggtcaac atggtggagc acgacacact tgtctactcc     120
   aaaaatatca aagatacagt ctcagaagac caaagggcaa ttgagacttt tcaacaaagg     180
   gtaatatccg gaaacctcct cggattccat tgcccagcta tctgtcactt tattgtgaag     240
   atagtggaaa aggaaggtgg ctcctacaaa tgccatcatt gcgataaagg aaaggccatc     300
   gttgaagatg cctctgccga cagtggtccc aaagatggac ccccacccac gaggagcatc     360
   gtggaaaaag aagacgttcc aaccacgtct tcaaagcaag tggattgatg tgataacatg     420
   gtggagcacg acacacttgt ctactccaaa aatatcaaag atacagtctc agaagaccaa     480
   agggcaattg agacttttca acaaagggta atatccggaa acctcctcgg attccattgc     540
   ccagctatct gtcactttat tgtgaagata gtggaaaagg aaggtggctc ctacaaatgc     600
   catcattgcg ataaaggaaa ggccatcgtt gaagatgcct ctgccgacag tggtcccaaa     660
   gatggacccc cacccacgag gagcatcgtg gaaaaagaag acgttccaac cacgtcttca     720
   aagcaagtgg attgatgtga tatctccact gacgtaaggg atgacgcaca atcccactat     780
   ccttcgcaag acccttcctc tatataagga agttcatttc atttggagag cagtttttaa     840
   ccatccttct actagacgta cactcgtaca tcctctagta tttcctttat ttatcttccc     900
   ataagcccct gcctttaacg tttaactctc gctatcgaca tggcattctt gaacgtttct     960
   gctgtcccca gctgtgcttt cgcacctgct ttcgcacccc acgccggtgc aagccccatt    1020
   gtgcccgaca gtttcccttg cgtaccccgt tattcggacg acatatccca tttccgttta 1080
   acgttgtcgc ttgacttctc tgtcccgcgc ccgctctttt tgaacgcgcg tgtgcacttg 1140
   agagcttcta ccgacaaccc cttaccatcg cttccccttg gtttccacgc tgaaaccttt 1200
   gttttggaat tgaacggcag ttcagctccc ttctctatac cgtcccgcca cattgacttc 1260
   gttgtgaacc ggcctttctc cgtgttccct actgaagtcc tctccgtcag ttcccttcga 1320
   acaccttcta ggttgttcgc tcttttatgc gactttttcc tttactgttc caaaccaggt 1380
   ccttgcgttg aaattgcttc tttttcaact ccaccaccct gtttagtttc gaactgcgtt 1440
   gcacaaattc ccacgcacgc cgaaatggaa agtattcgtt ttccaacgaa gactcttcca 1500
   gctggtcggc tcttacagtt ccacaaacga aaatacacga aacgacctga aactctaatc 1560
   atacacgaga gcggcttggc tctcaaaacc agcgcgttag gtgttacatc gaaacctaat 1620
   tcgcgcccta tcaccgtgaa atctgcttcc ggtgaaaaat atgaagctta cgagatctct 1680
   cgtaaagact tcgaacgctc ccgcaggagg caacaaactc ctcgcgttcg ttctcacaag 1740
   ccccggaaga taaataaagc tgtcgaacct ttcttctttc cagaagaacc aaagaaagac 1800
   aaacggaaga gagcttctct tccgactaaa gatgaaggtt tcattacttt tgggaccttg 1860
   cgctttccgc tttcggaaac ccctaaggaa gaacctcgtc ttcctaaatt tcgggaagtt 1920
   gaaattcctg tagtcaagaa acatgctgta ccagcagtag tttctaaacc agttcgaacg 1980
   tttagaccgg tcgctaccac tggcgctgag tatgtcaacg ctcggaccca gtgttcgcgc 2040
   cgtccaagaa atcatccgat tttacgcagt gcttcttata ctttcggttt taagaaaatg 2100
   ccgctccagc gtttcatgaa agaaaagaaa gattactacg tgaagcgctc taaggtcgtg 2160
   agtagttgta gtgtaactaa aagtcctttg gaagctttga ccagcatact aaagaactta 2220
   ccacggtatt cctacaactc agaacggtta aaattttacg atcattttat cggcgacgac 2280
   tttgaaattg aagtgcatcc gctgcgaggc ggcaaattaa gtgtgcttct tattttgcct 2340
   aagggcgaag cttactgcgt agtcactgcg gccacgccgc agtaccacgc tgctttaact 2400
   attgcgcgtg gcgatcgtcc tcgcgttggt gaacttctgc agtaccgacc cggcgaaggg 2460
   ttatgttacc tcgcccacgc tgctctttgt tgcgctcttc agaagcgcac ctttcgcgaa 2520
   gaagacttct tcgttggtat gtacccgacg aagtttgtct tcgctaaacg actcactgaa 2580
   aagttgggtc ctagtgctct caaacatcct gtgcgcggaa gacaagtctc tcgttcactc 2640
   ttccactgtg atgtggcttc tgctttttcg agcccctttt acagtttgcc gcgtttcatc 2700
   ggcggcgtgg aagaagaagc tcctgagatc acgtcgtctc ttaagcacaa ggcgatcgaa 2760
   tcggtctacg aacgtgtcag cattcacaaa gacaacttgt tggctcgtag cgtggaaaag 2820
   gatttgatcg actttaaaga cgaaatcaag tcgctgtcga aagaaaaacg ttccgttacc 2880
   gtccccttct atatggggga ggccgtacag agtggtttga cgcgcgcgta ccctcagttt 2940
   aatttgagtt tcactcacag tgtctattcg gaccaccctg cggcggccgg ttcccgtctg 3000
   ttggaaaacg aaactttagc ctcgatggct aaatcttcct tttctgatat cgggggctgc 3060
   ccactttttc acataaagag ggggagcaca gattaccacg tgtgcagacc gatttacgac 3120
   atgaaggacg ctcaacgaag agtttcgagg gaactccaag cgagagggct ggtggaaaac 3180
   ctttcccgcg aacaacttgt tgaagctcag gcgcgcgttt cggtgtgccc tcacactctc 3240
   ggcaattgta acgtgaaaag cgacgtgcta atcatggtgc aggtttacga cgcttctttg 3300
   aacgaaattg cgtcggcgat ggtcctaaag gaatcgaaag tcgcttacct caccatggtg 3360
   actccgggtg aacttctaga tgaacgtgaa gccttcgcta tcgacgcttt ggggtgcgac 3420
   gtcgtcgtgg acacccgccg agacatggtg cagtacaaat tcggctcatc ttgttactgc 3480
   cacaaactgt cgaatataaa gaatataatg ttgactcccg cattcacttt cagcgggaat 3540
   cttttttcgg tcgagatgta cgaaaaccga atgggcgtca actactacaa aatcactcgt 3600
   tcagcgtatt ctcctgaaat acgcggcgtt aagacgctgc ggtaccgccg agcctgcacc 3660
   gaggtcgtgc aagttaaact gccacgcttc gataaaactc taaagacgtt tctctccggg 3720
   tatgattaca tatatttgga tgcgaaattc gtctctcgag tcttcgatta cgtggttagc 3780
   aactgtagcg ttgttaacag taaaaccttc gagtgggtgt ggagttacat aaaatcaagt 3840
   aaatcgcgcg ttgtcataag tgggaaagtg atacatcgag atgtgcatat cgacctcaaa 3900
   cactctgaat gttttgcggc tgtgatgctc gcggttgggg tgcgttctcg cacaactact 3960
   gaatttttag cgaagaacct caattattac actggcgatg cttcgtgctt tgagactatc 4020
   cgtttcctct ttcgggagtg gagccggagg gcttacgcgg aaataaatcg cagttttcga 4080
   aagcttatga agagcattct ttccgctggg ttagattacg aatttctcga tcttgacaac 4140
   tcgctccaac acttgcttga atactcagag gttgaagtgc gcgtttcaat cgctcagaat 4200
   ggtgaggtgg attgcaacga agagaaccgt gttttgacgg agataatagc cgaagcagcg 4260
   gacaggaaat ccatcgctca aggtctcagc ggagcgttga gctccgttcc aacgcaaccg 4320
   agaggcggtc tgagaggggg tagtcgccga agtggagttt ttttccttta taacttggta 4380
   gaggaagtcg gaaatctttt cttttccgtc ggtgatgccg tgcggtttct tgttaagtta 4440
   tttaaaactt tttccgactc tcccatcttt cgggtcgttc ggatgttcct ggacttggcg 4500
   gaagcagctt caccgttcgt ttcggttgtt tctttgtgcg cgtggttgcg cgaagctgta 4560
   agcgctttct caggctgggt tgccgacaga acggtgtcgg aaagtgtaaa aacctttgta 4620
   aaccgtacgg taaaaaggtt tctaaacttt atgtctgcaa aaaccctgac gaaaaaattt 4680
   tttaggtttt tcttatccgc ttccgcctta gctaaaaccg ttgtgaggaa ggcgaaggtg 4740
   attttggaag cttactggga agtgtggttc gaatcgattc tttcggatag tggagagtat 4800
   agtgctgttg aattttgtag cagcgttgtg atcacactgt taacgaattc cggtcggctg 4860
   ctacctgggt ttagtccatc cgctgtcatt actgaagtac tgcttgacct agccacgaaa 4920
   atatccattg aagttcttct aaaacaaatt tctcctgtcg actcgacagc ttcttcggcc 4980
   ttatatcgcc gcgtcctaag cgagattctg tcgaattttc gcacaatggg cgaacacggg 5040
   atcttcacta aagtcttttt actctgcggg tttctacccg tgtttgttag gaagtgcgtc 5100
   gctctgtgcg ttcctggtga catggcgacg tacgcccggt ttctcgaata cggggtcgac 5160
   gatctcttct ttttgggaag gtccgtgaac tcgattaaga actacctatg tgtggttgct 5220
   gccgggttgg tggactccat tgtcgattcc gttgttttga aactttccgg tgtcgccaaa 5280
   gagcgagtgc ttggttttaa atcaaaaatc ataaaaaatt ttttaaacgt ctttaggaag 5340
   gcgaaagtcg ttacacgtac ttcttccagc acggacttgt cggaagacga atatttttcg 5400
   tgcgacgaaa gcaaacccgg cttgagaggc ggttcctcta ggttcacgct ttcgcgattg 5460
   cttgacatct ttttcaattt cctgaaaagt tcaaagctcg tcatagagaa tgcgtgcttt 5520
   tctgcttacg aaaggattga aaggaacatg aaactatact tttttccttt aaattcttcg 5580
   gaagaagaag ctcgccgctt aattcggtgc gcgggagact tcgactactt aagcgatagc 5640
   gctttcgacg aagatgaaat gttacggcaa gctttcgaac aatactattc cagtgacgac 5700
   gagagtgtaa cttacgatgg aaaacccaca gttctccgta gttacttgaa cgtgagtaga 5760
   aggttcttgg aaacgttctg taacggcccg aagtttttcg tcaaagtttc gaattacttt 5820
   aaagcgctgt atagtcgttt gctccgtgtt ctaccgtggg tagacagaaa tctttctgac 5880
   tcgccaggac ttaaaggagg taacgagaaa gctcttcttg cgaagttcct taaaacctgc 5940
   gtcataaccg cttgcgaatg tgtgtcgcag atatgctgcc tgcgtcttat tcgcttatgt 6000
   tgggggacac cagcgtgtgg tttagttagg ttattttaca taacttattc cggtactcgc 6060
   gtgttgtcgc gtgttgtggt cgcggtggct gtatgccctc ttttggtcag aaacgaactg 6120
   gatggcttga gtgatggatt aaccaacatg ggcgtttcgg tttttcgtcg cttgttcgtc 6180
   gcccttcggc gcgcgctttc ggcgtactct aattccgccc tacgacggaa gattttcgaa 6240
   ttcattttcg gaaacattca ccaccctttt gatgtcgcag taatcgaaac aaacgaagtt 6300
   gcgccggaac ctctttcacc ggaagtggac attgatgtcg actgcgactt tggttccgat 6360
   tcagaatcgg tttcttcaga tgaagtcgcg tcgattcccc gtccaggctt acacggtggg 6420
   aataggcgct cttccaactt tctaacctct ctcgtgaagg ttgtttttaa gttggctggg 6480
   cgcattccgc gtttgctttt ccgtttacgc aattttgtgg cgtactttgt tgaacgacgg 6540
   ctggcttcaa aaaggttgaa gacgttcatc ggtctggcca ggctgtttga taatttctca 6600
   ctcacttcgg tagtttacct tctccaagag tacgattcag tgctgaacgc atttatagac 6660
   gttgagctag tcctacttaa ctcgggtagc gtgaatgtgc tacctttagt ttcttgggtc 6720
   aggggatctc taacgaagtt agcggaagtg atcgttggtt caggcttcgc ttcgtttcta 6780
   ggaaggatgt gctgtcgtgt gtccgactgg tgctcctcgt cttcgaacgc cggttgtaac 6840
   tttatgagtc cggttcgtac gaaagggaag ttcgttcccc cttcgtcttc cggttcaacc 6900
   gcttcgatgt atgaacgtct cgaagctctc gagagcgata tccgcgaaca cgtgctttcc 6960
   acgtgtcgtg taggaagcga cgaagaagag gaaaggccga aagaagtgac agaaccagga 7020
   attgaacata cttctgaaga tgttgttccc attcgttcac actcgcaacc cttatctgga 7080
   ggtgaatgtt cgtattctga agatcgtgaa gagaatgaac gagcgaacct gttaccgcac 7140
   gttagcaaaa tcgtcagcga acgaaggggt ttggagaccg cccgtcgaaa caaacgtact 7200
   ctacgtggtg taagcgagtt tctcaacgct attaatacta gcaatgagca acctaggccg 7260
   ataatcgttg accactctcc tgaatctcgc gcgttgacca actccgtgag ggaattctat 7320
   tacctccagg aactcgctct ttttgagttg agttgcaaac ttcgtgagta ttacgatcaa 7380
   ttgaaggttg cgaattttaa cagacaagag tgtttgtgcg acaaagacga agacatgttt 7440
   gttctacgag ccggacaagg tgtagtttcc ggcagaaact cgaggttgcc tcttaagcat 7500
   ttcaaggatc acgaattttg ttttcgctct ggagggttgg tcccttacga cggtaccagc 7560
   agagtggaca ccatttttca cacgcaaacg aatttcgttt ccgcgaacgc gcttctttcg 7620
   ggctatctct cctatagaac tttcactttc actaatttga gcgctaacgt actgctgtac 7680
   gaagctcctc caggtggtgg aaagaccacg actttgataa aggttttttg tgaaactttt 7740
   tcaaaagtta attcgctgat tttaacggcg aataagagtt cgcgagaaga aatacttgcg 7800
   aaggtgaatc gcatcgtact tgacgaaggc gatacgcctc ttcagacgcg tgacaggatt 7860
   ttaactatcg attcttatct aatgaacaac agaggtttga cgtgtaaggt tttgtacctc 7920
   gatgagtgtt tcatggttca cgctggagct gctgtagctt gcatcgaatt caccaaatgc 7980
   gattcagcca tcttatttgg agacagcagg caaattcact acatagaccg taacgaattg 8040
   gatactgctg ttctttcaga tttgaaccgt tttgtcgatg atgaatcgag agtatatggt 8100
   gaagtctcat acaggtgtcc ttgggacgtt tgtgcttggt tgtcaacttt ctacccgaaa 8160
   actgtggcca ctaccaactt ggtttcagcc ggtcaatctt cgatgcaagt acgcgagatt 8220
   gaaagcgtag acgacgtcga atattccagt gaattcgtct acttgactat gttacagtca 8280
   gagaagaaag atctgctgaa atctttcggc aagaggtctc gttcgagcgt tgaaaaaccc 8340
   acggtcttaa cagtccatga agctcaaggt gaaacctacc gcaaagttaa cctcgtcaga 8400
   acgaaatttc aagaggacga tccttttcgt agcgagaacc acatcacagt ggccttgtct 8460
   aggcatgtcg agagtctgac ctattcggtc ctgagtagta aacgtgacga cgcaatagct 8520
   caagctatag cgaaggcgaa acaacttgtg gatgcctatc gcgtttaccc cacgtcattt 8580
   ggtgggagta ctcttgatat tagtgttaac ccttctacat ctgacaggag caaatgtaaa 8640
   gcttcctctg ccccttacga agttataaac agcttcttgg agagcgtggt tccgggcact 8700
   acttcagtag actttgggga cgtttccgaa gagatgggca ctcaggtttt tgagtccggt 8760
   gctgataacg ttgttattcg tgattccgca cctgttaaca agtcgacgga tcacgacccg 8820
   cagcgggttt agctcgattc gctcgcaggc gattcctaag aggaaaccgt cgctgcaaga 8880
   gaatttatac tcttatgagt cgcgtaatta caactttacc gtttgtgaac gtttttccgg 8940
   accgcaggag ttcggacagg cgatggcgat ggttatgttg gaacgaagct ttgacttaga 9000
   gaaagttgct aaagttagaa gcgatgtgat cgccataaca gaaaaagggg tgcgaacatg 9060
   gatgtcaaaa cgtgaacctt ctcagcttag ggctcttagt agtgacttac aaaagcctct 9120
   aaacttggaa gaggaaataa cgacttttaa gttgatggtt aagcgggacg cgaaagtcaa 9180
   actcgattcg tcgtgtttgg tgaaacaccc accagcgcag aatataatgt tccatcgcaa 9240
   ggcggtgaac gcgattttct cgccgtgttt cgacgagttt aaaaatagag tcattacctg 9300
   tacgaattca aatattgttt tctttaccga aatgactaac tctactctcg cgtcgatagc 9360
   gaaagagatg ctggggagcg aacacgttta caacgttggg gaaatagact tttcgaaatt 9420
   cgacaaatcc caagacgctt tcattaagtc gtttgaacga accttgtatt cagcgtttgg 9480
   tttcgacgaa gacctgcttg acgtgtggat gcaaggtgaa tacaccagca acgctacaac 9540
   tctggacggt caactttctt tttccgtcga caaccagagg aaatcgggcg cttctaatac 9600
   gtggattggt aattccatcg tgactcttgg cattttgagc atgttctatt acaccaatcg 9660
   atttaaggct cttttcgtgt ccggggacga ctctttgatt ttctccgaat ctcctattag 9720
   aaattcagcc gatgcgatgt gcacagaact cggttttgag actaagtttc tcactccgag 9780
   cgtcccgtat ttctgctcaa agtttttcgt tatgaccggt cacgacgttt tcttcgtgcc 9840
   cgacccttat aaacttttag tgaaattagg agcttctaag gatgaagtgg acgatgagtt 9900
   tctgtttgaa gtgttcacct cttttcgcga tttaacgaaa gatttagtcg atgaaagagt 9960
   gatcgaactc ttgacgcatt tggttcacag taagtacggg tacgaaagtg gtgatacgta 10020
   cgccgccctg tgtgctattc attgtattcg ttcaaacttt tcatcgttca agaaattgta 10080
   ccctaaagtt aagggctggg tcgttcacta cggtaaactg aagtttgtgc tgcgcaaatt 10140
   cgcgaactgt tttcgcgaga agtttgacac tgctttcggc gaagcgtact ttcttactta 10200
   cgacgaaact tgagactgtg tggtaactcg gttgttgttt tgtttgtttg tgtgtctgta 10260
   gtcctacttc gcggtgatgg gatccttaat taaggcgcgc ctgtacagct agctatggcc 10320
   ggcctctaga gaagtgacgg aggtgaccta cctgccgtgt aatttaagtc gtcacagagt 10380
   gacaacggca ccaagtggtg ctttgtgcgt atgtaaattg cgaagtgaaa aaatttttta 10440
   aaaaaatttt tcacactcct agcgaagtcc cgctaggaag aaaaaataag ggccgtcaag 10500
   acatgattca catgtcttct gatgagtccg tgaggacgaa acggcccggg gatcgttcaa 10560
   acatttggca ataaagtttc ttaagattga atcctgttgc cggtcttgcg atgattatca 10620
   tataatttct gttgaattac gttaagcatg taataattaa catgtaatgc atgacgttat 10680
   ttatgagatg ggtttttatg attagagtcc cgcaattata catttaatac gcgatagaaa 10740
   acagaatata gcgcgcaaac taggataaat tatcgcgcgc ggtgtcatct atgttactag 10800
   atcaagctta tcgataccgt cgacctcgag ggggggcccg gtaccaaaac caccccagta 10860
   cattaaaaac gtccgcaatg tgttattaag ttgtctaagc gtcaatttgt ttacaccaca 10920
   atatatcctg cca 10933
<210> 3
   <211> 236
   <212> DNA
   <213> Beet yellows virus
<400> 3
   cgtcagggta tgagttcctc ggggcagatt ttctaactgc gaccagcgtg tgtttgaacg 60
   atcacgaaaa agctatcgta ctacaggcct caagagctgc cattgataga gcagtctctt 120
   cgtcggtcga cgggaagatc gtcagtcttt tcgacctcgg tcgtcttagt taacacagtt 180
   actaaggttc cattttatta ttgcattgtt tttcatttag tgtaatcgta cttgag 236
<210> 4
   <211> 392
   <212> DNA
   <213> Beet yellow stunt virus
<400> 4
   aaccaaatgg tttaagggct ttttgcgcct ctttagaagg aatgtacctt tctgtagctc 60
   gactgggccc ggacgcgttc ggcactaggt ccgttgggaa gcgtggtgcg ccttcaggaa 120
   gcgagtattt aggcgccgat tttctgacct caacgtgtcc gttaatgtcc gaccacgatc 180
   gtgctgtagc tttgagtgca tctagaaacg cactcgaccg atctgcggcg tctcaaattg 240
   ataaaaagat ggttagtttg tacgacttcg gtaaggtagt gtatacctag cagcttgctt 300
   ctacctgaca cagttaagaa gcggcataaa tcgaagccaa accctaaatt ttgcaactcg 360
   atcaattgta acctagagcg aagtgcaatc at 392
<210> 5
   <211> 378
   <212> DNA
   <213> grapevine leafroll-associated virus 2
<400> 5
   gatgaaaagt ctcacgaaga agtacaaacg agtgaatggt ctgcgtgcgt tctgttgcgc      60
   gtgcgaagat ctatatctaa ccgtcgcacc aataatgtca gaacgcttta agactaaagc     120
   cgtagggatg aaaggtttgc ctgttggaaa ggaatactta ggcgccgact ttctttcggg     180
   aactagcaaa ctgatgagcg atcacgacag ggcggtctcc atcgttgcag cgaaaaacgc     240
   tgtcgatcgt agcgctttca cgggtgggga gagaaagata gttagtttgt atgatctagg     300
   gaggtactaa gcacggtgtg ctatagtgcg tgctataata ataaacacta gtgcttaagt     360
   cgcgcagaag aaaacgct                                                   378
<210> 6
   <211> 245
   <212> PRT
   <213> Artificial Sequence
<220>
   <223> Synthetic
<400> 6
   Met Gly Phe Val Leu Phe Ser Gln Leu Pro Ser Phe Leu Leu Val Ser
   1               5                   10                  15
   Thr Leu Leu Leu Phe Leu Val Ile Ser His Ser Cys Arg Ala Glu Ile
               20                  25                  30
   Val Leu Thr Gln Ser Pro Gly Thr Leu Ser Leu Ser Pro Gly Glu Arg
           35                  40                  45
   Ala Thr Leu Ser Cys Arg Ala Ser Gln Ser Val Ser Tyr Ser Ser Leu
       50                  55                  60
   Ala Trp Tyr Gln Gln Lys Pro Gly Gln Ala Pro Ser Leu Leu Ile Tyr
   65                  70                  75                  80
   Gly Ala Ser Ser Arg Ala Thr Gly Ile Pro Asp Arg Phe Ser Gly Ser
                   85                  90                  95
   Gly Ser Gly Pro Asp Phe Thr Leu Thr Ile Ser Arg Leu Glu Pro Glu
               100                 105                 110
   Asp Phe Ala Val Tyr Tyr Cys Gln His Tyr Gly Asn Ser Pro Tyr Thr
           115                 120                 125
   Phe Gly Gln Gly Thr Lys Leu Glu Ile Lys Arg Thr Val Ala Ala Pro
       130                 135                 140
   Ser Val Phe Ile Phe Pro Pro Ser Asp Glu Gln Leu Lys Ser Gly Thr
   145                 150                 155                 160
   Ala Ser Val Val Cys Leu Leu Asn Asn Phe Tyr Pro Arg Glu Ala Lys
                   165                 170                 175
   Val Gln Trp Lys Val Asp Asn Ala Leu Gln Ser Gly Asn Ser Gln Glu
               180                 185                 190
   Ser Val Thr Glu Gln Asp Ser Lys Asp Ser Thr Tyr Ser Leu Ser Ser
           195                 200                 205
   Thr Leu Thr Leu Ser Lys Ala Asp Tyr Glu Lys His Lys Val Tyr Ala
       210                 215                 220
   Cys Glu Val Thr His Gln Gly Leu Ser Ser Pro Val Thr Lys Ser Phe
   225                 230                 235                 240
   Asn Arg Gly Glu Cys
                   245
<210> 7
   <211> 738
   <212> DNA
   <213> Artificial Sequence
<220>
   <223> synthetic
<400> 7
   atgggatttg ttctcttttc acaattgcct tcatttcttc ttgtctctac acttctctta      60
   ttcctagtaa tatcccactc ttgccgtgcc gaaattgtgc ttactcagtc tccaggaact     120
   ctttctcttt ctccaggtga aagggctact ctttcttgca gggcttctca gtctgtgtct     180
   tactcttctc ttgcttggta tcagcaaaag ccaggacaag ctccatctct tcttatttac     240
   ggtgcttctt ctagggctac tggtattcca gataggttct ctggatctgg atctggtcca     300
   gatttcactc ttactatttc taggcttgag ccagaggatt tcgctgttta ctactgccag     360
   cactacggaa attctccata cactttcgga cagggaacta agcttgagat taagaggact     420
   gtggctgctc catctgtgtt tattttccca ccatctgatg agcaacttaa gtctggaact     480
   gcttctgttg tgtgccttct taacaacttc tacccaaggg aagctaaggt tcagtggaaa     540
   gtggataacg ctcttcagtc tggaaactct caagagtctg tgactgagca ggattctaag     600
   gattcaactt actctctttc ttctactctt actttgtcta aggctgatta cgagaagcac     660
   aaggtttacg cttgcgaagt tactcatcag ggactttctt ctccagtgac taagtctttc     720
   aacaggggag agtgctga                                                  738
<210> 8
   <211> 479
   <212> PRT
   <213> Artificial Sequence
<220>
   <223> Synthetic
<400> 8
   Met Gly Phe Val Leu Phe Ser Gln Leu Pro Ser Phe Leu Leu Val Ser
   1               5                   10                  15
   Thr Leu Leu Leu Phe Leu Val Ile Ser His Ser Cys Arg Ala Gln Val
               20                  25                  30
   Gln Leu Val Gln Ser Gly Ala Glu Val Lys Lys Pro Gly Ala Ser Val
           35                  40                  45
   Lys Val Ser Cys Lys Ala Ser Gly Tyr Thr Phe Thr Ser Asn Ala Ile
       50                  55                  60
   Gln Trp Val Arg Gln Ala Pro Gly Gln Arg Leu Glu Trp Val Gly Trp
   65                  70                  75                  80
   Ile Asn Gly Gly Asp Gly Asn Thr Lys Tyr Ser Gln Lys Phe Gln Gly
                   85                  90                  95
   Arg Val Thr Ile Ser Arg Asp Ile Ser Ala Ser Thr Ala Tyr Met Glu
               100                 105                 110
   Leu Ser Ser Leu Arg Ser Glu Asp Thr Ala Val Tyr Tyr Cys Ala Arg
           115                 120                 125
   His Arg Leu Gln Arg Gly Gly Phe Asp Pro Trp Gly Gln Gly Thr Leu
       130                 135                 140
   Val Thr Val Ser Ser Ala Ser Thr Lys Gly Pro Ser Val Phe Pro Leu
   145                 150                 155                 160
   Ala Pro Ser Ser Lys Ser Thr Ser Gly Gly Thr Ala Ala Leu Gly Cys
                   165                 170                 175
   Leu Val Lys Asp Tyr Phe Pro Glu Pro Val Thr Val Ser Trp Asn Ser
               180                 185                 190
   Gly Ala Leu Thr Ser Gly Val His Thr Phe Pro Ala Val Leu Gln Ser
           195                 200                 205
   Ser Gly Leu Tyr Ser Leu Ser Ser Val Val Thr Val Pro Ser Ser Ser
       210                 215                 220
   Leu Gly Thr Gln Thr Tyr Ile Cys Asn Val Asn His Lys Pro Ser Asn
   225                 230                 235                 240
   Thr Lys Val Asp Lys Arg Val Glu Pro Lys Ser Cys Asp Lys Thr His
                   245                 250                 255
   Thr Cys Pro Pro Cys Pro Ala Pro Glu Leu Leu Gly Gly Pro Ser Val
               260                 265                 270
   Phe Leu Phe Pro Pro Lys Pro Lys Asp Thr Leu Met Ile Ser Arg Thr
           275                 280                 285
   Pro Glu Val Thr Cys Val Val Val Asp Val Ser His Glu Asp Pro Glu
       290                 295                 300
   Val Lys Phe Asn Trp Tyr Val Asp Gly Val Glu Val His Asn Ala Lys
   305                 310                 315                 320
   Thr Lys Pro Arg Glu Glu Gln Tyr Asn Ser Thr Tyr Arg Val Val Ser
                   325                 330                 335
   Val Leu Thr Val Leu His Gln Asp Trp Leu Asn Gly Lys Glu Tyr Lys
               340                 345                 350
   Cys Lys Val Ser Asn Lys Ala Leu Pro Ala Pro Ile Glu Lys Thr Ile
           355                 360                 365
   Ser Lys Ala Lys Gly Gln Pro Arg Glu Pro Gln Val Tyr Thr Leu Pro
       370                 375                 380
   Pro Ser Arg Glu Glu Met Thr Lys Asn Gln Val Ser Leu Thr Cys Leu
   385                 390                 395                 400
   Val Lys Gly Phe Tyr Pro Ser Asp Ile Ala Val Glu Trp Glu Ser Asn
                   405                 410                 415
   Gly Gln Pro Glu Asn Asn Tyr Lys Thr Thr Pro Pro Val Leu Asp Ser
               420                 425                 430
   Asp Gly Ser Phe Phe Leu Tyr Ser Lys Leu Thr Val Asp Lys Ser Arg
           435                 440                 445
   Trp Gln Gln Gly Asn Val Phe Ser Cys Ser Val Met His Glu Ala Leu
       450                 455                 460
   His Asn His Tyr Thr Gln Lys Ser Leu Ser Leu Ser Pro Gly Lys
   465                 470                 475
<210> 9
   <211> 1440
   <212> DNA
   <213> Artificial Sequence
<220>
   <223> Synthetic
<400> 9
   atgggatttg ttctcttttc acaattgcct tcatttcttc ttgtctctac acttctctta      60
   ttcctagtaa tatcccactc ttgccgtgcc caggttcagc ttgttcaatc tggtgctgag     120
   gttaagaaac ctggtgcttc tgttaaggtg tcatgcaagg cttcaggata cactttcact     180
   tctaacgcta ttcagtgggt taggcaagct ccaggacaaa gacttgaatg ggttggctgg     240
   attaacggtg gagatggaaa cactaagtac tctcagaagt tccagggaag ggttacaatt     300
   tctagggata tttctgcttc tactgcttac atggaacttt cttctcttag atctgaggat     360
   actgctgtgt attactgcgc taggcataga cttcaaaggg gaggatttga tccttgggga     420
   cagggaactc ttgtgactgt gtcatctgct tcaactaagg gaccatctgt gtttccactt     480
   gctccatctt ctaagtctac ttcaggtgga actgctgctc ttggatgcct tgtgaaggat     540
   tacttcccag agccagttac tgtttcttgg aactctggtg ctcttacttc tggtgttcac     600
   actttcccag ctgtgcttca gtcatctgga ctttactcac tttcttcagt ggtgactgtg     660
   ccttcttctt ctcttggaac tcagacttac atctgcaacg tgaaccacaa gccatctaac     720
   acaaaagtgg ataagagggt ggagccaaag tcttgcgata agactcatac ttgtccacca     780
   tgtccagctc cagaacttct tggaggacct tctgtgttcc ttttcccacc aaagccaaag     840
   gatactctta tgatttctag gactccagag gttacatgcg ttgtggttga tgtgtctcat     900
   gaggatccag aggtgaagtt caactggtac gtggatggtg ttgaggttca caacgctaag     960
   actaagccaa gggaagagca gtacaactct acttacaggg ttgtgtctgt gcttactgtg    1020
   cttcaccagg attggcttaa cggaaaggaa tacaagtgca aggtgtcaaa caaggctctt    1080
   ccagctccaa ttgaaaagac tatttctaag gctaagggac aacctagaga gccacaggtt    1140
   tacactcttc caccatctag ggaagagatg actaagaacc aggtgtcact tacttgcttg    1200
   gtgaagggat tctacccatc tgatattgct gttgagtggg agtctaatgg acaaccagag    1260
   aacaactaca agactactcc accagtgctt gattctgatg gatctttctt cctttactct    1320
   aagcttactg tggataagtc taggtggcag cagggaaatg ttttctcttg ctctgtgatg    1380
   catgaggctc ttcacaatca ctacactcag aagtctttgt ctctttctcc tggaaagtga    1440
<210> 10
   <211> 777
   <212> PRT
   <213> Artificial Sequence
<220>
   <223> Synthetic
<400> 10
   Met Gly Phe Val Leu Phe Ser Gln Leu Pro Ser Phe Leu Leu Val Ser
   1               5                   10                  15
   Thr Leu Leu Leu Phe Leu Val Ile Ser His Ser Cys Arg Ala Glu Val
               20                  25                  30
   Lys Gln Glu Asn Arg Leu Leu Asn Glu Ser Glu Ser Ser Ser Gln Gly
           35                  40                  45
   Leu Leu Gly Tyr Tyr Phe Ser Asp Leu Asn Phe Gln Ala Pro Met Val
       50                  55                  60
   Val Thr Ser Ser Thr Thr Gly Asp Leu Ser Ile Pro Ser Ser Glu Leu
   65                  70                  75                  80
   Glu Asn Ile Pro Ser Glu Asn Gln Tyr Phe Gln Ser Ala Ile Trp Ser
                   85                  90                  95
   Gly Phe Ile Lys Val Lys Lys Ser Asp Glu Tyr Thr Phe Ala Thr Ser
               100                 105                 110
   Ala Asp Asn His Val Thr Met Trp Val Asp Asp Gln Glu Val Ile Asn
           115                 120                 125
   Lys Ala Ser Asn Ser Asn Lys Ile Arg Leu Glu Lys Gly Arg Leu Tyr
       130                 135                 140
   Gln Ile Lys Ile Gln Tyr Gln Arg Glu Asn Pro Thr Glu Lys Gly Leu
   145                 150                 155                 160
   Asp Phe Lys Leu Tyr Trp Thr Asp Ser Gln Asn Lys Lys Glu Val Ile
                   165                 170                 175
   Ser Ser Asp Asn Leu Gln Leu Pro Glu Leu Lys Gln Lys Ser Ser Asn
               180                 185                 190
   Ser Arg Lys Lys Arg Ser Thr Ser Ala Gly Pro Thr Val Pro Asp Arg
           195                 200                 205
   Asp Asn Asp Gly Ile Pro Asp Ser Leu Glu Val Glu Gly Tyr Thr Val
       210                 215                 220
   Asp Val Lys Asn Lys Arg Thr Phe Leu Ser Pro Trp Ile Ser Asn Ile
   225 230 235 240
   His Glu Lys Lys Gly Leu Thr Lys Tyr Lys Ser Ser Pro Glu Lys Trp
   245 250 255
   Ser Thr Ala Ser Asp Pro Tyr Ser Asp Phe Glu Lys Val Thr Gly Arg
   260 265 270
   Ile Asp Lys Asn Val Ser Pro Glu Ala Arg His Pro Leu Val Ala Ala
   275 280 285
   Tyr Pro Ile Val His Val Asp Met Glu Asn Ile Ile Leu Ser Lys Asn
   290 295 300
   Glu Asp Gln Ser Thr Gln Asn Thr Asp Ser Glu Thr Arg Thr Ile Ser
   305 310 315 320
   Lys Asn Thr Ser Thr Ser Arg Thr His Thr Ser Glu Val His Gly Asn
   325 330 335
   Ala Glu Val His Ala Ser Phe Phe Asp Ile Gly Gly Ser Val Ser Ala
   340 345 350
   Gly Phe Ser Asn Ser Asn Ser Ser Thr Val Ala Ile Asp His Ser Leu
   355 360 365
   Ser Leu Ala Gly Glu Arg Thr Trp Ala Glu Thr Met Gly Leu Asn Thr
   370 375 380
   Ala Asp Thr Ala Arg Leu Asn Ala Asn Ile Arg Tyr Val Asn Thr Gly
   385 390 395 400
   Thr Ala Pro Ile Tyr Asn Val Leu Pro Thr Thr Ser Leu Val Leu Gly
   405 410 415
   Lys Asn Gln Thr Leu Ala Thr Ile Lys Ala Lys Glu Asn Gln Leu Ser
   420 425 430
   Gln Ile Leu Ala Pro Asn Asn Tyr Tyr Pro Ser Lys Asn Leu Ala Pro
   435 440 445
   Ile Ala Leu Asn Ala Gln Asp Asp Phe Ser Ser Thr Pro Ile Thr Met
   450 455 460
   Asn Tyr Asn Gln Phe Leu Glu Leu Glu Lys Thr Lys Gln Leu Arg Leu
   465 470 475 480
   Asp Thr Asp Gln Val Tyr Gly Asn Ile Ala Thr Tyr Asn Phe Glu Asn
   485 490 495
   Gly Arg Val Arg Val Asp Thr Gly Ser Asn Trp Ser Glu Val Leu Pro
   500 505 510
   Gln Ile Gln Glu Thr Thr Ala Arg Ile Ile Phe Asn Gly Lys Asp Leu
   515 520 525
   Asn Leu Val Glu Arg Arg Ile Ala Ala Val Asn Pro Ser Asp Pro Leu
   530 535 540
   Glu Thr Thr Lys Pro Asp Met Thr Leu Lys Glu Ala Leu Lys Ile Ala
   545 550 555 560
   Phe Gly Phe Asn Glu Pro Asn Gly Asn Leu Gln Tyr Gln Gly Lys Asp
   565 570 575
   Ile Thr Glu Phe Asp Phe Asn Phe Asp Gln Gln Thr Ser Gln Asn Ile
   580 585 590
   Lys Asn Gln Leu Ala Glu Leu Asn Ala Thr Asn Ile Tyr Thr Val Leu
   595 600 605
   Asp Lys Ile Lys Leu Asn Ala Lys Met Asn Ile Leu Ile Arg Asp Lys
   610 615 620
   Arg Phe His Tyr Asp Arg Asn Asn Ile Ala Val Gly Ala Asp Glu Ser
   625 630 635 640
   Val Val Lys Glu Ala His Arg Glu Val Ile Asn Ser Ser Thr Glu Gly
   645 650 655
   Leu Leu Leu Asn Ile Asp Lys Asp Ile Arg Lys Ile Leu Ser Gly Tyr
   660 665 670
   Ile Val Glu Ile Glu Asp Thr Glu Gly Leu Lys Glu Val Ile Asn Asp
   675 680 685
   Arg Tyr Asp Met Leu Asn Ile Ser Ser Leu Arg Gln Asp Gly Lys Thr
   690 695 700
   Phe Ile Asp Phe Lys Lys Tyr Asn Asp Lys Leu Pro Leu Tyr Ile Ser
   705 710 715 720
   Asn Pro Asn Tyr Lys Val Asn Val Tyr Ala Val Thr Lys Glu Asn Thr
   725 730 735
   Ile Ile Asn Pro Ser Glu Asn Gly Asp Thr Ser Thr Asn Gly Ile Lys
               740                 745                 750
   Lys Ile Leu Ile Phe Ser Lys Lys Gly Tyr Glu Ile Gly Lys Leu His
           755                 760                 765
   His His His His His Lys Asp Glu Leu
       770                 775
<210> 11
   <211> 2334
   <212> DNA
   <213> Artificial Sequence
<220>
   <223> Synthetic
<400> 11
   atgggatttg ttctcttttc acaattgcct tcatttcttc ttgtctctac acttctctta      60
   ttcctagtaa tatcccactc ttgccgtgcc gaggtgaagc aagagaatag gcttcttaat     120
   gagtctgagt catcttctca gggattgctt ggttactact tctctgatct taatttccag     180
   gctcctatgg tggttacttc ttctactact ggtgatcttt ctattccttc ttctgagctt     240
   gagaatattc cttctgagaa tcagtacttc cagtctgcta tttggagtgg tttcattaag     300
   gtgaagaaat ctgatgagta cactttcgct acttcagctg ataatcatgt gactatgtgg     360
   gtggacgatc aggaggtgat caataaggct tctaattcta ataagattag gcttgagaag     420
   ggaaggcttt accagattaa gattcagtac cagagggaaa atcctactga gaagggtctt     480
   gatttcaagt tgtactggac tgattcacag aataagaaag aagtgatttc ttctgataat     540
   cttcagcttc ctgagcttaa gcagaagtca tctaattcta ggaagaagag gtctacttct     600
   gctggtccta ctgttcctga tagggataat gatggtattc ctgattctct tgaggtggag     660
   ggttacactg tggatgtgaa gaataagagg actttccttt ctccttggat ttctaatatt     720
   catgagaaga agggtcttac taagtacaag tcatctcctg agaagtggtc tactgcttct     780
   gatccttact ctgatttcga gaaggtgaca ggaaggattg ataagaatgt gtctcctgag     840
   gctagacatc ctcttgttgc tgcttaccct attgtgcatg tggatatgga gaatattatt     900
   ctttctaaga atgaggatca gtctactcag aatactgatt ctgagactag gactatttct     960
   aagaatactt ctacttctag gactcatact tctgaagtgc atggaaatgc tgaagttcat    1020
   gcttctttct tcgatattgg tggttctgtg tctgctggtt tctctaattc aaattcttct    1080
   actgtggcta ttgatcattc tctttctctt gctggtgaaa ggacttgggc tgagactatg    1140
   ggacttaata ctgcagatac tgctaggctt aatgctaata ttagatacgt gaatactggt    1200
   actgctccta tctacaatgt gcttcctact acttctttgg tgcttggaaa gaatcagact    1260
   cttgctacta ttaaggctaa agagaatcag ctttctcaga ttcttgctcc taacaattac     1320
   tacccttcta agaatcttgc tccaattgct cttaatgctc aggatgattt ctcttctact     1380
   cctattacta tgaattacaa tcagttcctt gagcttgaaa agactaagca gcttaggctt     1440
   gatactgatc aggtgtacgg taatattgct acttacaatt tcgagaatgg tagagtgaga     1500
   gtggatactg gttctaattg gagtgaggtg ttgcctcaga ttcaagagac tactgctagg     1560
   attattttca atggaaagga tcttaatctt gtggagagaa ggattgctgc tgtgaatcct     1620
   tctgatcctc ttgagactac taagcctgat atgactctta aagaggctct taagattgct     1680
   ttcggtttca atgagcctaa tggaaatctt cagtaccagg gaaaggatat tactgagttc     1740
   gatttcaatt tcgatcagca gacttctcag aacattaaga accagctagc tgagttgaat     1800
   gctactaata tctacactgt gttggataag attaagttga atgcaaagat gaatattctt     1860
   attagggata agaggttcca ttacgatagg aacaatattg ctgtgggtgc tgatgagtct     1920
   gttgtgaaag aggctcatag ggaagttatc aattcttcaa ctgagggact tcttcttaat     1980
   attgataagg atattaggaa gattctttct ggttacattg tggagattga ggatactgag     2040
   ggtcttaaag aagtgatcaa tgatagatac gatatgttga atatttcttc tcttaggcag     2100
   gatggaaaga ctttcattga tttcaaaaag tacaatgata agttgcctct ttacatttct     2160
   aatcctaatt acaaagtgaa tgtgtacgct gtgacaaaag agaacactat tatcaatcca     2220
   tctgagaatg gtgatacttc tactaatggt attaagaaga ttttgatttt ctctaagaag     2280
   ggttacgaga ttggaaagct tcaccaccat catcatcata aggatgaact ttga           2334
<210> 12
   <211> 441
   <212> PRT
   <213> Artificial Sequence
<220>
   <223> Synthetic
<400> 12
   Met Gly Phe Val Leu Phe Ser Gln Leu Pro Ser Phe Leu Leu Val Ser
   1               5                   10                  15
   Thr Leu Leu Leu Phe Leu Val Ile Ser His Ser Cys Arg Ala Asn Asn
               20                  25                  30
   Asp Phe Cys Lys Pro Ser Ser Leu Asn Ser Glu Ile Ser Gly Phe Ile
           35                  40                  45
   Gly Tyr Lys Cys Asn Phe Ser Asn Glu Gly Val His Asn Leu Lys Pro
       50                  55                  60
   Asp Met Arg Glu Arg Arg Ser Ile Phe Cys Thr Ile His Ser Tyr Phe
   65                  70                  75                  80
   Ile Tyr Asp Lys Ile Arg Leu Ile Ile Pro Lys Lys Ser Ser Ser Pro
                   85                  90                  95
   Glu Phe Lys Ile Leu Pro Glu Lys Cys Phe Gln Lys Val Tyr Thr Asp
               100                 105                 110
   Tyr Glu Asn Arg Val Glu Thr Asp Ile Ser Glu Leu Gly Leu Ile Glu
           115                 120                 125
   Tyr Glu Ile Glu Glu Asn Asp Thr Asn Pro Asn Tyr Asn Glu Arg Thr
       130                 135                 140
   Ile Thr Ile Ser Pro Phe Ser Pro Lys Asp Ile Glu Phe Phe Cys Phe
   145                 150                 155                 160
   Cys Asp Asn Thr Glu Lys Val Ile Ser Ser Ile Glu Gly Arg Ser Ala
                   165                 170                 175
   Met Val His Val Arg Val Leu Lys Tyr Pro His Asn Ile Leu Phe Thr
               180                 185                 190
   Asn Leu Thr Asn Asp Leu Phe Thr Tyr Leu Pro Lys Thr Tyr Asn Glu
           195                 200                 205
   Ser Asn Phe Val Ser Asn Val Leu Glu Val Glu Leu Asn Asp Gly Glu
       210                 215                 220
   Leu Phe Val Leu Ala Cys Glu Leu Ile Asn Lys Lys Cys Phe Gln Glu
   225                 230                 235                 240
   Gly Lys Glu Lys Ala Leu Tyr Lys Ser Asn Lys Ile Ile Tyr His Lys
                   245                 250                 255
   Asn Leu Thr Ile Phe Lys Ala Pro Phe Tyr Val Thr Ser Lys Asp Val
               260                 265                 270
   Asn Thr Glu Cys Thr Cys Lys Phe Lys Asn Asn Asn Tyr Lys Ile Val
           275                 280                 285
   Leu Lys Pro Lys Tyr Glu Lys Lys Val Ile His Gly Cys Asn Phe Ser
       290                 295                 300
   Ser Asn Val Ser Ser Lys His Thr Phe Thr Asp Ser Leu Asp Ile Ser
   305                 310                 315                 320
   Leu Val Asp Asp Ser Ala His Ile Ser Cys Asn Val His Leu Ser Glu
                   325                 330                 335
   Pro Lys Tyr Asn His Leu Val Gly Leu Asn Cys Pro Gly Asp Ile Ile
               340                 345                 350
   Pro Asp Cys Phe Phe Gln Val Tyr Gln Pro Glu Ser Glu Glu Leu Glu
           355                 360                 365
   Pro Ser Asn Ile Val Tyr Leu Asp Ser Gln Ile Asn Ile Gly Asp Ile
       370                 375                 380
   Glu Tyr Tyr Glu Asp Ala Glu Gly Asp Asp Lys Ile Lys Leu Phe Gly
   385                 390                 395                 400
   Ile Val Gly Ser Ile Pro Lys Thr Thr Ser Phe Thr Cys Ile Cys Lys
                   405                 410                 415
   Lys Asp Lys Lys Ser Ala Tyr Met Thr Val Thr Ile Asp Ser Ala His
               420                 425                 430
   His His His His His Lys Asp Glu Leu
           435                 440
<210> 13
   <211> 1326
   <212> DNA
   <213> Artificial Sequence
<220>
   <223> Synthetic
<400> 13
   atgggattcg tgcttttctc tcagcttcct tctttccttc ttgtgtctac tcttcttctt      60
   ttccttgtga tttctcactc ttgtagggct aacaacgatt tctgcaagcc atcttctctt     120
   aactctgaga tttctggatt cattggatac aagtgcaact tctctaacga gggtgttcac     180
   aaccttaagc cagatatgag agagagaaga tcaattttct gcactattca ctcttacttc     240
   atttacgata agattaggct tattattcca aagaagtcat cttctccaga gttcaagatt     300
   cttccagaga agtgcttcca gaaggtgtac actgattacg agaacagggt ggagactgat     360
   atttctgagc ttggacttat tgagtacgag attgaagaga acgatacaaa cccaaactac     420
   aacgagagga ctattactat ttctccattc tctccaaagg atattgagtt cttctgcttc     480
   tgcgataaca ctgagaaagt gatttcttct attgagggaa gatcagctat ggttcatgtg     540
   agggtgttga agtacccaca caacattctt ttcactaacc ttactaacga tcttttcact     600
   tacttgccaa agacttacaa cgagtctaac ttcgtgtcta acgtgcttga ggtggagctt      660
   aatgatggtg agttgttcgt tcttgcttgc gagcttatta acaagaagtg tttccaagag      720
   ggaaaagaga aggctcttta caagtctaac aagattattt accacaagaa ccttactatt      780
   ttcaaggctc cattctacgt gacttctaag gatgtgaaca ctgagtgcac ttgcaagttc      840
   aagaacaaca actacaagat tgtgcttaag ccaaagtacg agaagaaagt gattcacgga      900
   tgcaacttct catctaacgt gtcatctaag cacactttca ctgattctct tgatatttct      960
   cttgtggatg attctgctca catttcttgc aacgtgcacc tttctgagcc aaagtacaac     1020
   caccttgtgg gacttaattg cccaggtgat attattccag attgcttctt ccaggtttac     1080
   caaccagagt ctgaagaact tgagccatct aacattgtgt accttgattc tcagattaac     1140
   attggagata ttgagtacta cgaggatgct gagggtgatg ataagattaa gttgttcgga     1200
   attgtgggat ctattccaaa gactacttct ttcacttgca tctgcaagaa ggataagaaa     1260
   tctgcttaca tgactgtgac tattgattca gctcatcacc atcaccacca caaggatgag     1320
   ctttga                                                                1326
<210> 14
   <211> 355
   <212> PRT
   <213> Artificial Sequence
<220>
   <223> Synthetic
<400> 14
   Met Gly Phe Val Leu Phe Ser Gln Leu Pro Ser Phe Leu Leu Val Ser
   1               5                   10                  15
   Thr Leu Leu Leu Phe Leu Val Ile Ser His Ser Cys Arg Ala Ala Pro
               20                  25                  30
   Ala Asp Asn Thr Val Asn Ile Lys Thr Phe Asp Lys Val Lys Asn Ala
           35                  40                  45
   Phe Gly Asp Gly Leu Ser Gln Ser Ala Glu Gly Thr Phe Thr Phe Pro
       50                  55                  60
   Ala Asp Val Thr Thr Val Lys Thr Ile Lys Met Phe Ile Lys Asn Glu
   65                  70                  75                  80
   Cys Pro Asn Lys Thr Cys Asp Glu Trp Asp Arg Tyr Ala Asn Val Tyr
                   85                  90                  95
   Val Lys Asn Lys Thr Thr Gly Glu Trp Tyr Glu Ile Gly Arg Phe Ile
               100                 105                 110
   Thr Pro Tyr Trp Val Gly Thr Glu Lys Leu Pro Arg Gly Leu Glu Ile
           115                 120                 125
   Asp Val Thr Asp Phe Lys Ser Leu Leu Ser Gly Asn Thr Glu Leu Lys
       130                 135                 140
   Ile Tyr Thr Glu Thr Cys Leu Ala Lys Gly Arg Glu Tyr Ser Val Asp
   145                 150                 155                 160
   Phe Asp Ile Val Tyr Gly Thr Pro Asp Tyr Lys Tyr Ser Ala Val Val
                   165                 170                 175
   Pro Val Ile Gln Tyr Asn Lys Ser Ser Ile Asp Gly Val Pro Tyr Gly
               180                 185                 190
   Lys Ala His Thr Leu Gly Leu Lys Lys Asn Ile Gln Leu Pro Thr Asn
           195                 200                 205
   Thr Glu Lys Ala Tyr Leu Arg Thr Thr Ile Ser Gly Trp Gly His Ala
       210                 215                 220
   Lys Pro Tyr Asp Ala Gly Ser Arg Gly Cys Ala Glu Trp Cys Phe Arg
   225                 230                 235                 240
   Thr His Thr Ile Ala Ile Asn Asn Ala Asn Thr Phe Gln His Gln Leu
                   245                 250                 255
   Gly Ala Leu Gly Cys Ser Ala Asn Pro Ile Asn Asn Gln Ser Pro Gly
               260                 265                 270
   Asn Trp Ala Pro Asp Arg Ala Gly Trp Cys Pro Gly Met Ala Val Pro
           275                 280                 285
   Thr Arg Ile Asp Val Leu Asn Asn Ser Leu Thr Gly Ser Thr Phe Ser
       290                 295                 300
   Tyr Glu Tyr Lys Phe Gln Ser Trp Thr Asn Asn Gly Thr Asn Gly Asp
   305                 310                 315                 320
   Ala Phe Tyr Ala Ile Ser Ser Phe Val Ile Ala Lys Ser Asn Thr Pro
                   325                 330                 335
   Ile Ser Ala Pro Val Val Thr Asn Asp Tyr Lys Asp Asp Asp Asp Lys
               340                 345                 350
   Asp Glu Leu
   355
<210> 15
   <211> 1068
   <212> DNA
   <213> Artificial Sequence
<220>
   <223> Synthetic
<400> 15
   atgggtttcg tgctgttctc tcagcttcca tctttccttt tggtgtctac ccttcttctg      60
   ttccttgtga tttctcattc ttgcagagct gctccagctg ataacaccgt gaacattaag     120
   accttcgata aggtgaagaa cgctttcggt gatggtcttt ctcaatctgc tgagggaact     180
   tttaccttcc ctgctgatgt gactaccgtt aagaccatca agatgttcat caagaacgag     240
   tgccctaaca agacttgtga tgagtgggat aggtacgcta atgtgtacgt gaagaacaag     300
   actactggtg agtggtatga gatcggtaga ttcattactc cttactgggt gggaactgag     360
   aagcttccta gaggtcttga gattgatgtg accgatttca agtctctgct gtctggtaat     420
   accgagctta agatctacac cgagacttgt cttgctaagg gtagagagta ctccgttgat     480
   ttcgatattg tgtacggaac ccctgattac aagtactcag ctgttgttcc tgtgatccag     540
   tacaacaagt ctagcattga tggtgtgcca tacggtaagg ctcatactct tggtctgaag     600
   aagaacattc agttgcctac taacaccgag aaggcttatc ttaggaccac tatttctggt     660
   tggggtcatg ctaagcctta tgatgctggt tctagaggtt gtgctgagtg gtgttttagg     720
   actcatacca ttgctatcaa caacgctaac actttccagc atcagcttgg tgctcttggt     780
   tgttctgcta accctattaa caaccagtct cctggtaatt gggctcctga tagagctggt     840
   tggtgtcctg gtatggctgt tcctactagg attgatgtgc tgaacaactc tcttaccggt     900
   tctacattca gctacgagta caagttccag tcttggacta acaacggtac taacggtgat 960
   gctttctacg ctattagctc tttcgtgatc gctaagtcta atacccctat ttctgctcct 1020
   gtggtgacca atgattacaa ggatgatgat gataaggatg agctttag                 1068